# EUROPEAN PATENT APPLICATION

(11) **EP 2 090 227 A1**
(43) Date of publication of application: **19.08.2009**
(21) Application number: 07831065.3
(22) Date of filing: 01.11.2007
(51) Int. Cl.: A61B 5/151, A61B 5/157, G01N 27/28, G01N 27/327, G01N 27/416

(54) **BIOSENSOR CARTRIDGE, BIOSENSOR DEVICE, SPECIMEN SAMPLING METHOD, MANUFACTURING METHOD FOR BIOSENSOR CARTRIDGE, AND NEEDLE-INTEGRATED SENSOR**

(30) Priority: 10.11.2006 JP 2006304972; 20.11.2006 JP 2006313466; 22.02.2007 JP 2007043034
(71) Applicant: National Institute Of Advanced Industrial Science, Tokyo 100-8921 (JP); Sumitomo Electric Industries, Ltd., Chuo-ku Osaka-shi Osaka 540-0041 (JP)
(72) Inventor: FUJIMURA, Tsuyoshi, Tsukuba-shi Ibaraki 305-8562 (JP); NAKAMURA, Hideaki, Tsukuba-shi Ibaraki 305-8562 (JP); ISHIKAWA, Tomoko, Tsukuba-shi Ibaraki 305-8562 (JP); GOTOH, Masao, Tsukuba-shi Ibaraki 305-8562 (JP); KARUBE, Isao, Tsukuba-shi Ibaraki 305-8562 (JP); KITAMURA, Takahiko, Osaka-shi Osaka 554-0024 (JP); KAIMORI, Shingo, Osaka-shi Osaka 554-0024 (JP); HARADA, Akira, Osaka-shi Osaka 554-0024 (JP); NAKAJIMA, Hiroto, Osaka-shi Osaka 554-0024 (JP); HAYAMI, Hiroshi, Osaka-shi Osaka 554-0024 (JP); HOSOYA, Toshifumi, Osaka-shi Osaka 554-0024 (JP)
(74) Representative: Setna, Rohan P.
(86) International application number: PCT/JP2007/071331
(87) International publication number: WO 2008/056598

(57) **Abstract**

A biosensor cartridge capable of performing puncturing in an exact position, and easily collecting and measuring a sample of a puncture hole without requiring the operation of bringing a sample collection opening close to the puncture hole is provided. When tip portion 11a of biosensor chip body 11 is pushed against a subject M, elastic body 20 protruding from a tip portion 11a of biosensor chip body 11 is compressed, and puncturing tool 12 for puncturing protrudes. Thus, the subject M can be punctured. Additionally, if a pushing force is weakened, puncturing tool 12 is extracted from the subject M by the restoring force of elastic body 20, and the sample R flows out of the puncture hole. Additionally, cover member 19 is provided around biosensor chip body 11. Thus, when puncturing is performed by thin plate-shaped biosensor cartridge 10, biosensor chip body 11 can be prevented from deforming, and puncturing can be accurately performed in a desired puncturing position.

## Description

### TECHNICAL FIELD

The present invention relates to a biosensor cartridge, a biosensor device, a sample collecting method, a manufacturing method of a biosensor cartridge, and a needle integral sensor which conducts measurement and analysis of a chemical substance using a reagent contained in a hollow reaction portion of, for example, a biosensor chip.

### RELATED ART

In related art, for example, a biosensor chip which detects the concentration of glucose in blood is known (for example, refer to Patent Document 1).
Fig. 22 is an exploded perspective view showing a glucose sensor described in Patent Document 1. As shown in Fig. 22, glucose sensor 1100 that is a biosensor has counter electrode 1101 and working electrode 1102. Counter electrode 1101 assumes a hollow needle shape which is half-cut in its longitudinal direction, and tip portion 1103 thereof is obliquely cut in the shape of an injection needle for easy puncture. Insulating layers 1104 and 1104' which_generally serve also as an adhesive layer, for example, epoxy resin adhesive, silicone-based adhesive, or glass is applied to the half-cut face, and working electrode 1102 is attached to the half-cut face via insulating layers 1104 and 1104'. Working electrode 1102 is a flat-plate-shaped member where glucose oxidase (GOD) is immobilized, and is bonded to counter electrode 1101 with a face where GOD is immobilized being directed inward.
Accordingly, a subject is punctured by tip portion 1103 of needlelike counter electrode 1101 to collect blood, and the reaction between the collected blood and immobilized GOD 1105 is detected by working electrode 1102, thereby performing the quantitative determination of glucose.

Additionally, a biosensor in which a biosensor chip and a lancet are integrated is disclosed (for example, refer to Patent Document 2).
Fig. 23A is a perspective view showing a sensor described in Patent Document 2, and Fig. 23B is an exploded perspective view of the sensor. As shown in Fig. 23, lancet integral sensor 1110 has chip body 1111, lancet 1113, and protective cover 1115. Chip body 1111 has cover 1111a and substrate 1111b openably and closably, and an inner surface of cover 1111a is formed with internal space 1112. Internal space 1112 assumes a shape which can house lancet 1113 movably.

Needle 1114 provided at the tip of lancet 1113 is able to protrude and retract from opening 1112a formed at a front end of internal space 1112 of chip body 1111 with movement of lancet 1113. By pressing both side surfaces of chip body 1111 with fingers, and pressing protrusions 1113a of lancet 1113, lancet 1113 can be fixed to chip body 1111, and performs puncture in the fixed state. Protective cover 1115 has tube portion 1115a into which needle 1114 is inserted and fitted, and tube portion 1115a can also be housed inside chip body 1111 with movement of needle 1114. Accordingly, in the state before use, protective cover 1115 is put on needle 1114 to protect needle 1114 and prevent a user from being damaged accidentally. In addition, substrate 1111b is provided with a pair of electrode terminals 1116, and these terminals can be electrically connected to a measuring device (not shown).

Accordingly, at the time of use, protective cover 1115 is removed, lancet 1113 is pushed to make needle 1114 protrude from chip body 1111, and both side surfaces of chip body 1111 are pressed with fingers to fix needle 1114. After a subject is punctured in this state, needle 1114 is housed in chip body 1111, and opening 1112a provided at the front end of chip body 1111 is brought close to a puncture hole, whereby blood which has flowed out is collected.
Patent Document 1: Unexamined Japanese Patent Application Publication No. Hei2-120655
Patent Document 2: Pamphlet of PCT International Patent Publication No. 02/056769

### DISCLOSURE OF THE PRESENT INVENTION

### PROBLEMS TO BE SOLVED BY THE PRESENT INVENTION

However, since needlelike counter electrode 1101 and working electrode 1102 are formed so as to be stuck together in glucose sensor 1100 described in Patent Document 1, the diameter of a puncturing needle becomes nearly equal to the width of glucose sensor 1100, and becomes large. For this reason, there is a problem that the amount of blood sampled increases, and pain during puncturing becomes large, and consequently, user's burden becomes large.
Additionally, in the lancet integral sensor 1110 described in Patent Document 2, a puncture drive mechanism is provided within internal space 1112. Therefore, there is a problem that a complicated puncture mechanism is needed, and manufacture is complicated.

Additionally, the lancet integral sensor described in Patent Document 2 drives needle 1114 within internal space 1112 which houses a sample. Therefore, the size of internal space 1112 becomes a size required for driving of needle 1114, and the amount of blood required for measurement increases more than that of a measurement kit in which a sensor and a puncturing needle are separate. This makes a needle thick or makes a needle pierce a subject deeply, thereby increasing the pain at the time of collecting of the subject.

Meanwhile, a sensor in which a cavity containing blood is provided separately from a lancet storage space within a sensor body is also suggested in Patent Document 2. In this case, since the size of the cavity can be set independently of the lancet, it can be considered that it becomes possible to reduce the amount of blood collected. However, even though the piercing position by the lancet and the inlet of the cavity are disposed in different positions, a mechanism for effectively containing blood to the surface of skin discharged blood adhered to the needle to the cavity is not disclosed at all.

The present invention is made in view of the aforementioned problems, and the object of the present invention is to provide a biosensor device, a sample collecting method, a biosensor cartridge which can perform puncturing in an exact position and can easily collect and measure a sample in a puncture hole without requiring the operation of bringing the sample collection opening close to the puncture hole, a method of manufacturing a biosensor cartridge which can manufacture a biosensor cartridge easily, and a needle integral sensor which is formed as a needle integral sensor in which a puncturing needle is fixed to a sensor and which can introduce blood discharged to a skin surface into a cavity even in a position where the puncturing position of the needle, and an inlet of the cavity are separated from each other.

### MEANS FOR SOLVING THE PROBLEMS

In order to achieve the aforementioned object, according to a first aspect of the present invention, there is provided a biosensor cartridge including:
a biosensor chip,
a puncturing tool fixed to a portion of the biosensor chip and protruding from the tip of the biosensor chip,
an elastic body provided at the tip of the biosensor chip, and
a cover member provided around the biosensor chip.

In the biosensor cartridge configured in this way, when the tip portion of the biosensor chip is pushed against a subject, the elastic body provided at the tip portion of the biosensor chip is compressed, and the puncturing tool protrudes. Thus, the subject can be punctured. Additionally, if a pushing force is weakened, the puncturing tool is extracted from the subject by the restoring force of the elastic body, and the sample flows out of the puncture hole. Additionally, the cover member is provided around the biosensor chip. Thus, when puncturing is performed by the thin plate-shaped biosensor cartridge, the biosensor chip can be prevented from deforming. That is, the thickness of only the biosensor cartridge is minute. Thus, there is a possibility that the biosensor chip may deform during puncturing, and thus, puncture cannot be performed in a predetermined position, and a sample cannot be collected, there is a possibility that the puncturing direction may be unexpectedly inclined with respect to a subject's skin, or there is possibility that a pain is involved. In a case where the cover member is provided, puncturing can be accurately performed in a desired puncturing position, and the needle can puncture the subject's skin substantially perpendicularly thereto. Further, since the cover member is provided even when the biosensor cartridge is mounted on driving unit, the area of contact with the connector is large, and the mounting is easy. Moreover, since the area of contact with the elastic body becomes large, the cover member can be easily integrated with the elastic body, and the problem that the elastic body is separated from the biosensor cartridge can be solved.
If the shape of the cover member is cylindrical or prismatic, the area of contact with the elastic body can be increased, thereby securing the strength of the cover member.

Additionally, according to a second aspect of the present invention, there is provided the biosensor cartridge of the first aspect, wherein
the elastic body and the cover member are bonded together.

In the biosensor cartridge configured in this way, the elastic body and the cover member are bonded together. Thus the possibility that positions of the elastic body and the biosensor cartridge may deviate during puncturing and puncturing may be performed in a wrong position is low, and puncturing can be reliably performed.
Additionally, even a small amount of sample can be reliably collected within the biosensor chip by the bonding.

Additionally, according to a third aspect of the present invention, there is provided the biosensor cartridge of the first or second aspect, wherein
the elastic body forms an enclosed space so as to contain a sample collection opening provided at the tip of the biosensor chip and a puncture hole formed in a subject by the puncturing tool.

In biosensor cartridge configured in this way, since the puncture hole, and the sample collection opening provided at the tip of the biosensor chip are contained in the enclosed space formed by the elastic body, even a small amount of sample can be sucked from the puncture hole, and be introduced into the sample collection opening.

Additionally, according to a fourth aspect of the present invention, there is provided the biosensor cartridge according to any one of the first to third aspects, wherein
the elastic body has stickiness.

In the biosensor chip configured in this way, since the elastic body has stickiness, adhesion with the cover member improves, deviation from a puncturing position is prevented, the enclosed space can be reliably formed, and a sample can be reliably introduced into a sample introduction port from a puncture hole.

Additionally, according to a fifth aspect of the present invention, there is provided the biosensor cartridge according to any one of the first to fourth aspects, wherein
puncturing is performed by compressing the elastic body to make the puncturing tool protrude from the tip of the elastic body.

In the biosensor cartridge configured in this way, when the tip portion of the biosensor chip body is pushed against a subject, the elastic body provided at the tip portion of the biosensor chip is compressed, and the puncturing tool protrudes. Thus, the subject can be easily punctured. Moreover, the puncturing tool is prevented from protruding from the tip face of the elastic body before use, so that protection of the puncturing tool and protection of a user can be achieved. Additionally, even when being discarded after use, the puncturing tool is prevented from protruding from the tip face of the elastic body, so that the tool can be safely and properly disposed.

Additionally, according to a sixth aspect of the present invention, there is provided a biosensor device including:
the biosensor cartridge of any one of the first to fifth aspects, and
a measuring instrument which is connected to detecting electrodes of the biosensor cartridge and obtains information on a sample which has been collected.

In the biosensor device configured in this way, a sample is collected by the aforementioned biosensor cartridge. Thus, puncturing and collecting can be performed by a series of operation, collecting of a sample can be easily and reliably ensured without being required to align the sample collection opening of the biosensor chip with the puncture hole unlike a related art. Additionally, information on a sample is transmitted to the measuring instrument via the detecting electrodes, so that measurement can be easily performed in a short time, and a burden on a subject can be reduced.

Additionally, according to a seventh aspect of the present invention, there is provided the biosensor device of the sixth aspect, further including:
a driving unit for puncturing, and
a connector for attaching the biosensor cartridge, the connector being attached to the driving unit.

In the biosensor device configured in this way, the connector for connecting the biosensor cartridge is attached to the driving unit. For example, puncturing can be performed by a drive mechanism like a spring or a motor. Thus, puncturing can be performed in a short time, and a burden on a user can be reduced.

Additionally, according to an eighth aspect of the present invention, there is provided the biosensor device, wherein
the cover member and the connector fit to each other.

In the biosensor device configured in this way, as the cover member and the connector fit to each other, the cover member, and the connector can be reliably attached to the driving unit.

Additionally, according to a ninth aspect of the present invention, there is provided a sample collecting method including the steps of:
performing puncturing using the biosensor cartridge according to any one of the first to seventh aspects, and
collecting a sample from the sample collection opening using a suction unit connected to the biosensor cartridge.

In the sample collecting method configured in this way, puncturing is performed using the biosensor cartridge, and a sample is collected from the sample collection opening provided at the tip of the biosensor cartridge by the suction unit connected to the biosensor cartridge. Thus, even a small amount of sample can also be reliably collected.

In order to achieve the aforementioned object, according to a tenth aspect of the present invention, there is provided a method of manufacturing a biosensor cartridge including a biosensor chip having a sample collection opening at a tip face thereof and a puncturing tool fixed to a tip portion of the biosensor chip and having a tip portion protruding therefrom,
the method including the step of:
making a first fitting portion provided in a cover member having the puncturing tool integrally and a second fitting portion provided in the biosensor chip fit to each other.

In the method of manufacturing a biosensor cartridge configured in this way, when a biosensor cartridge in which a puncturing tool is fixed to a tip portion of the biosensor chip and from which a tip portion protrudes is manufactured, a first fitting portion provided in the cover member, and a second fitting portion provided in the biosensor chip are made to fit to each other, and thereby, both are integrated. Thus, assembling is easy, integration can be firmly achieved, and the puncturing tool can be easily attached to the biosensor chip.

Additionally, according to an eleventh aspect of the present invention, there is provided the method of manufacturing a biosensor cartridge, wherein
the first fitting portion is a fitting hole provided in the cover member, and
the second fitting portion is the biosensor chip body which makes the biosensor chip fit to the fitting hole.

In the method of manufacturing a biosensor cartridge configured in this way is the method according to the first feature of the above invention in which a fitting hole as the first fitting portion provided in the cover member and the biosensor chip body as the second fitting portion provided in the biosensor chip are made to fit to each other, and thereby, both are integrated. Thus, assembling is easy, integration can be firmly achieved, and the puncturing tool can be easily attached to the biosensor chip.

Additionally, according to a twelfth aspect of the present invention, there is provided the method of manufacturing a biosensor cartridge of the tenth or eleventh aspect, wherein
the first fitting portion is either a protrusion or a recess provided along an insertion direction in the cover member, and
the second fitting portion is the other of a protrusion and a recess which fits to either the protrusion or the recess provided in the biosensor chip.

In the method of manufacturing a biosensor cartridge configured in this way, in the tenth or eleventh feature of the above invention, a protrusion or a recess as the first fitting portion provided in the cover member, and a recess or a protrusion as the second fitting portion provided in the biosensor chip are made to fit to each other, and thereby, both are integrated. Thus, assembling is easy, integration can be firmly achieved, and the puncturing tool can be easily attached to the biosensor chip.

Additionally, according to a thirteenth aspect of the present invention, there is provided the method of manufacturing a biosensor cartridge of any one of the tenth to twelfth aspects, wherein
the biosensor chip and the puncturing tool are fixed together by joining the biosensor chip and the cover member with an adhesive.

In the method of manufacturing a biosensor cartridge configured in this way, the cover member and the biosensor chip can be easily fixed together by making the cover member and the biosensor chip fit to each other, and joining them with an adhesive. Thereby, the puncturing tool provided in the cover member can be easily attached to the biosensor chip.

Additionally, according to a fourteenth aspect of the present invention, there is provided the method of manufacturing a biosensor cartridge of any one of the tenth to thirteenth aspects, wherein
the cover member includes a locking claw in the vicinity of a rear end in the insertion direction, and
the biosensor chip and the puncturing tool are fixed together by locking a stepped portion provided in the biosensor chip.

In the method of manufacturing a biosensor cartridge configured in this way, when the biosensor chip is inserted into the cover member, the stepped portion provided at the rear end of the biosensor chip is locked by the locking claw provided at the rear end of the cover member. Thus, the biosensor chip and the cover member can be reliably fixed, and the puncturing tool can be attached to the biosensor chip.

In the needle integral sensor in which the puncturing needle is fixed to the sensor, in order to perform piercing required to discharge a liquid sample, such as blood, from a subject to a skin surface, the puncturing needle is fixed in a protruding state. For this reason, although the positional relationship that the tip of puncturing needle and the inlet of a sample reaction space are separated from each other is inevitably obtained, this can be solved by providing a flow passage through which a liquid sample can flow from the tip of the puncturing needle to the inlet of a sample reaction space. Thus, the present invention has variously examined attaching a flow passage forming body which forms a flow passage through which a liquid sample can flow from the tip of the puncturing needle to the inlet of a sample reaction space, and the attachment structure thereof, and has completed the present invention.

That is, there is provided a needle integral sensor including:
a needle integral sensor body integrally having a puncturing needle which discharges a liquid sample from a subject, a reaction portion which contains the liquid sample, and a detecting portion which detects the result of the reaction portion, and
a flow passage forming body having an attachment portion in which a recess into which the tip of the needle integral sensor body is inserted is recessed, and a flow passage portion which forms a flow passage from an abutting portion of the subject to the reaction portion and in which the puncturing needle is inserted into the flow passage, wherein
the flow passage forming body is fastened and fixed to the needle integral sensor body by a fastener.
In such a needle integral sensor, insertion operation of the flow passage forming body in a loose fitting relation and attaching and fixing operation of the flow passage forming body by a fastener after the insertion are completed, and deviation of attaching operation is little, and it is easy to cope with mass production.

Although the material of the flow passage forming body is not particularly limited, the material may be a rigid body, may be an elastic body, and may be a viscoelastic body. However, preferably, at least the flow passage portion is formed of an elastic body or a viscoelastic body. By making the flow passage portion of an elastic body or a viscoelastic body, by deformation and restoration of the flow passage portion, the puncturing needle is able to protrude and retract from the flow passage forming body, and pullout from a subject is automatically performed after puncturing.

The shape of the flow passage forming body and the type of the fastener are not particularly limited, but are suitably selected according to the shape of the tip of a sensor body to be inserted, workability, the attachment position relationship between the flow passage forming body and the needle integral sensor body, or the like.

For example, in a case where an inserted portion of the needle integral sensor body is substantially columnar, preferably, the attachment portion is cylindrical into which the inserted portion can be inserted, and one which fastens the recess from its whole outer periphery and reduces the diameter thereof is used as the fastener.

Additionally, in a case where the shape of the inserted portion of the needle integral sensor body is a square shape or an asymmetrical shape, or in a case where the position where the flow passage forming body should be attached for is determined, a fastener which fastens the sensor body from a predetermined direction is suitable. The fastener which fastens the sensor body from a predetermined direction includes, for example, a screw, a pin, etc. Since the flow passage forming body can be attached and fixed not by fastening the whole recess, but simply by fastening from a predetermined direction, the shape of the inserted portion of the needle integral sensor body and the shape of the recess just have to be shapes fastened by the fastener. Accordingly, the shape of the recess and the shape of the inserted portion of the needle integral sensor body which is fitted may not be a similar shape. Even if the inserted portion of the sensor body has a shape other than a substantially circular shape, the recess can be formed in a simple shape, such as a substantially circular shape or square shape, and can be formed as a structure where a gap is formed between the recess and the sensor body. Additionally, since the attachment position of the flow passage forming body can be uniquely determined by providing the sensor body with a screw hole or a pin hole corresponding to a fastener, such as a screw or a pin, deviation of an attachment position in attachment operation of the flow passage forming body can be prevented. Accordingly, it is suitable as an attachment structure in a case where the positional relationship between the inlet of the reaction portion and the flow passage is determined.

### ADVANTAGE OF THE PRESENT INVENTION

According to the biosensor cartridge and sample collecting method of the present invention, the tip portion of the biosensor chip is provided with the elastic body. Thus, when the tip portion of the biosensor chip is pushed against a subject, the subject can be punctured by compressing the elastic body and making the puncturing tool protruding from the tip of the elastic body, and when a pushing force is weakened, the puncturing tool is pulled out of the subject by the restoring force of the elastic body, and a sample flows out of the puncture hole. Additionally, the cover member is provided around the biosensor chip. Thus, when puncturing is performed by a thin plate-shaped biosensor cartridge, the biosensor chip can be prevented from deforming, and puncturing can be accurately performed in a desired puncturing position. Moreover, since the cover member is provided even when the biosensor cartridge is mounted on driving unit, the contact area is large, and the mounting is easy. Moreover, since the area of contact with the elastic body increases, setting to the elastic body is easy, and the problem that the elastic body is separated from the biosensor cartridge can be solved.
Additionally, according to the manufacturing method of a biosensor cartridge of the present invention, the first fitting portion provided in the cover member, and the second fitting portion provided in the biosensor chip are made to fit to each other, and thereby, both are integrated. Thus, assembling is easy, integration can be firmly achieved, and the puncturing tool provided in the cover member can be easily attached to biosensor chip.
Additionally, the needle integral sensor of the present invention includes a flow passage forming body which can guide a liquid sample discharged by puncturing to the inlet of the reaction portion disposed so as to be separated from the tip of the puncturing needle, and the flow passage forming body is fastened and fixed by fitting operation, or fastening operation by a fastener. Thus, the collecting amount of a liquid sample required for measurement can be reduced without complicating the structure of the needle integral sensor, and manufacturing processes.

### BRIEF DESCRIPTION OF THE DRAWINGS

Fig. 1A is a sectional view of essential portions showing a first embodiment according to a biosensor cartridge of the present invention. Fig. 1B is a sectional view showing the first embodiment according to the biosensor cartridge of the present invention.
Fig. 2 is an exploded perspective view showing an example of a cover member.
Fig. 3A is a perspective view showing an example of a suction unit used for a sample collecting method according to the present invention. Fig. 3B is a sectional view.
Fig. 4 is a configuration diagram showing a first embodiment according to a biosensor device of the present invention.
Figs. 5A to 5C are explanatory views showing blood collecting operation using the biosensor device of Fig. 4.
Fig. 6A is an explanatory views showing a modification of the first embodiment according to the biosensor cartridge in the first embodiment of the present invention. Fig. 6B is an explanatory views showing a modification of the first embodiment according to the biosensor cartridge of the present invention.
Fig. 7A is a sectional view showing a second embodiment according to the biosensor cartridge of the present invention. Fig. 7B is a sectional view of a B-B position in Fig. 7A showing the second embodiment according to the biosensor cartridge of the present invention.
Fig. 8A is a plan view of a biosensor chip. Fig. 8B is a side view of the biosensor chip. Fig. 8C is a front end view of the biosensor chip.
Fig. 9 is an exploded perspective view showing an example of the cover member.
Fig. 10A is a perspective view showing an example of the suction unit. Fig. 10B is a sectional view showing an example of the suction unit.
Fig. 11 is a configuration diagram showing an example of a biosensor device in a second embodiment of the present invention.
Fig. 12 is a sectional view of the cover member and biosensor chip of Embodiment 1.
Fig. 13 is a sectional view of the cover member and biosensor chip of Embodiment 2.
Figs. 14A to 14F are a plan view, a side view, a sectional view, and the like showing Embodiment 3.
Fig. 15 is a sectional view showing the configuration of a needle integral sensor of one embodiment of the present invention.
Figs. 16A and 16B are a plan view (Fig. 16A) and a sectional view (Fig. 16B) showing the configuration of a sensor used for the needle integral sensor of the embodiment of Fig. 15.
Fig. 17 is a perspective view of a flow passage forming body used for the needle integral sensor of the embodiment of Fig. 15.
Fig. 18 is a schematic diagram showing the configuration of one embodiment of a measuring device mounted with the needle integral sensor of the embodiment of Fig. 15.
Fig. 19 is a sectional view showing the configuration of a needle integral sensor of another embodiment of the present invention.
Fig. 20 is a sectional view showing another embodiment of a fastener used in the present invention.
Fig. 21 is a sectional view showing the configuration of a needle integral sensor of another embodiment of the present invention.

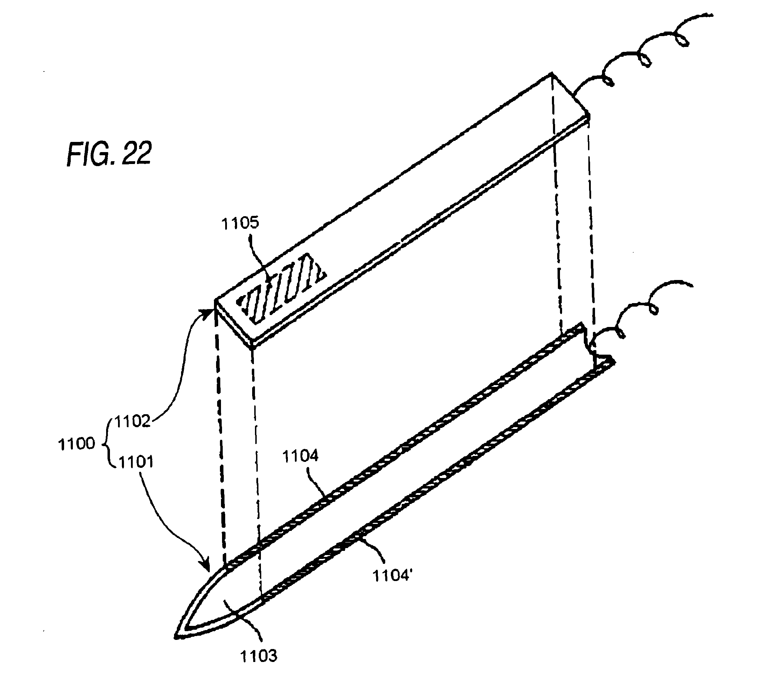

Fig. 22 is an exploded perspective view showing a biosensor chip of related art.

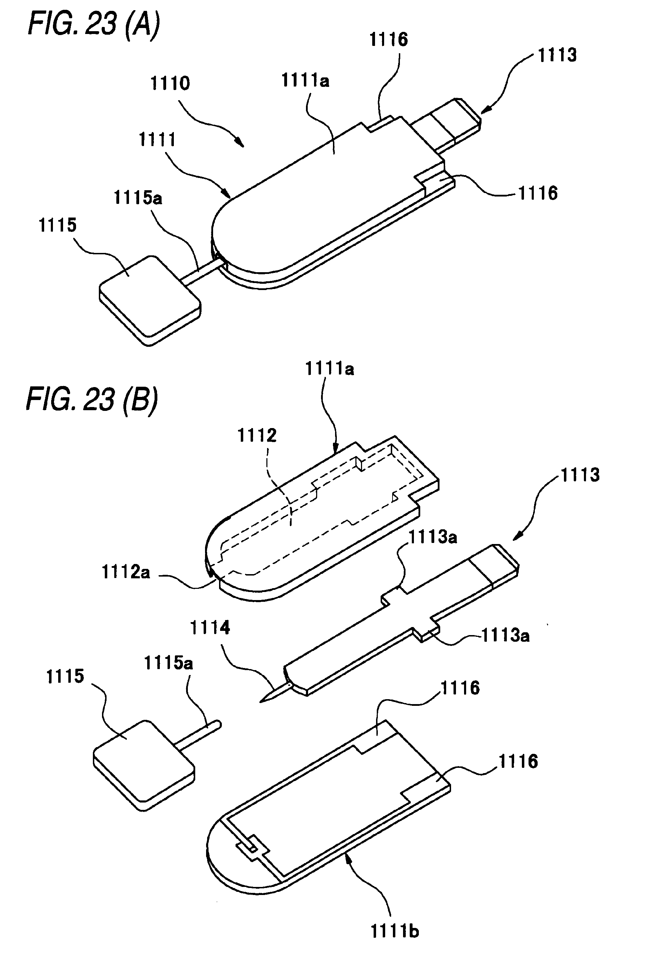

Fig. 23A is a perspective view showing the biosensor chip of the related art. Fig. 23B is an exploded perspective view showing the biosensor chip of the related art.

### BRIEF DESCRIPTION OF REFERENCE NUMERALS AND SIGNS

10, 110: BIOSENSOR CARTRIDGE
11: BIOSENSOR CHIP
11a, 111a: TIP PORTION
12, 112: PUNCTURING TOOL
12a, 112a: TIP
13, 113: SAMPLE COLLECTION OPENING
18a, 18b: DETECTING EELCTRODE
19, 130: COVER MEMBER
20: ELASTIC BODY
21: SURFACE TOUCHING SUBJECT (TIP FACE)
23: ENCLOSED SPACE
24: GLUING AGENT
30: BIOSENSOR DEVICE
31: MEASURING INSTRUMENT
40: DRIVING UNIT
41: CONNECTOR
42: SPRING
50: SUCTION UNIT
R: BLOOD (SAMPLE)
M: SUBJECT
111: BIOSENSOR CHIP BODY (SECOND FITTING PORTION)
111c: RECESS
119: STEPPED PORTION
131: FITTING HOLE (FIRST FITTING PORTION)
134: PROTRUSION
135: ADHESIVE
136: LOCKING CLAW
201: DETECTING PORTION
202: PUNCTURING NEEDLE
203: REACTION PORTION
203a: REACTION PORTION INLET
210: NEEDLE INTEGRAL SENSOR BODY
220, 220', 230: FLOW PASSAGE FORMING BODY
221, 221', 231: RECESS
222, 222', 232: FLOW PASSAGE
225: C RING
227, 228: FASTENER
234: ADJUSTING SCREW

### PREFERRED EMBODIEMTNS FOR CARRYING OUT THE PRESENT INVENTION

Hereinafter, a first embodiment according to the present invention will be described in detail with reference to the drawings.

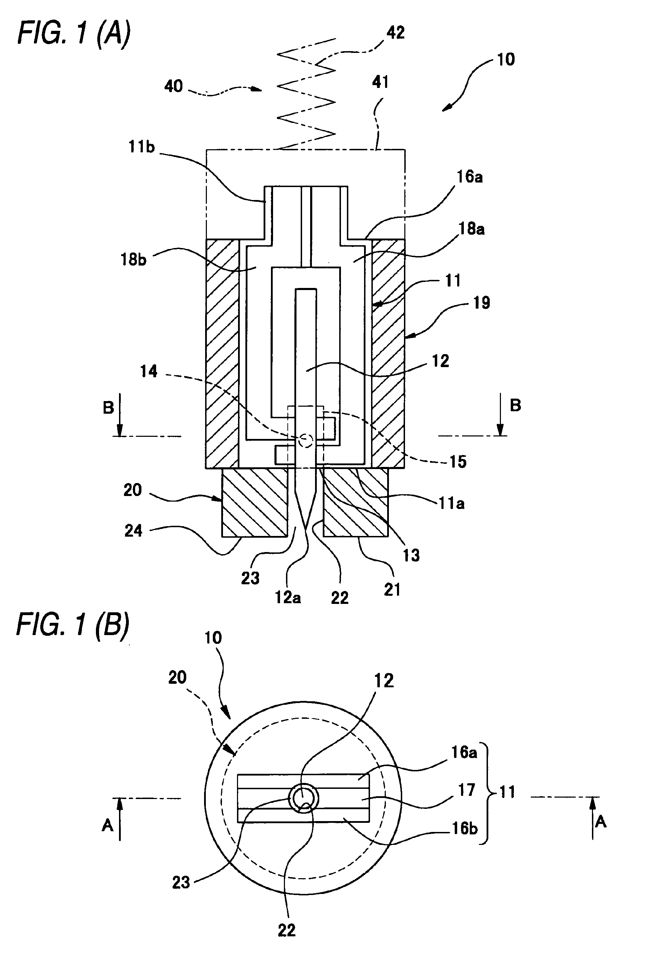

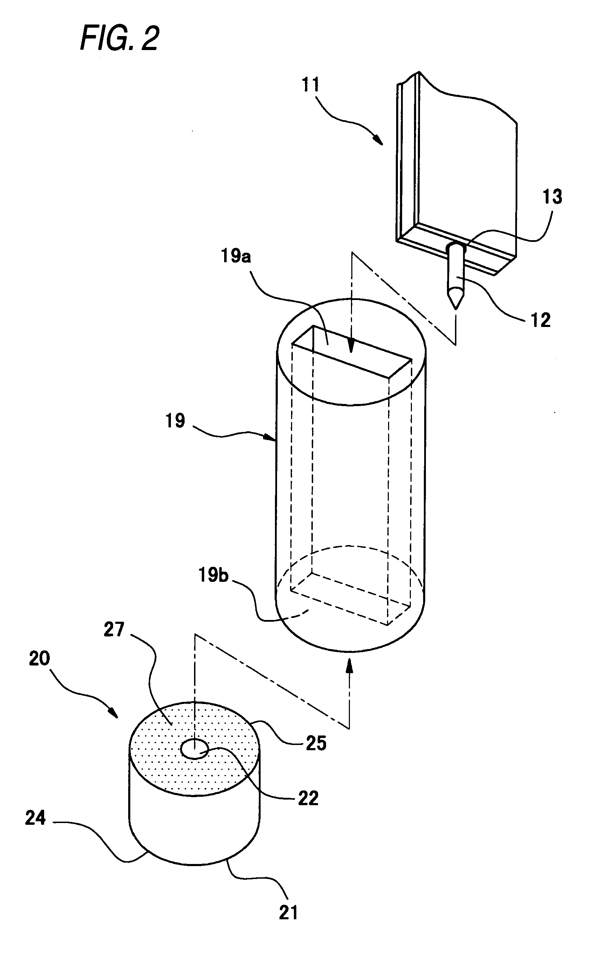

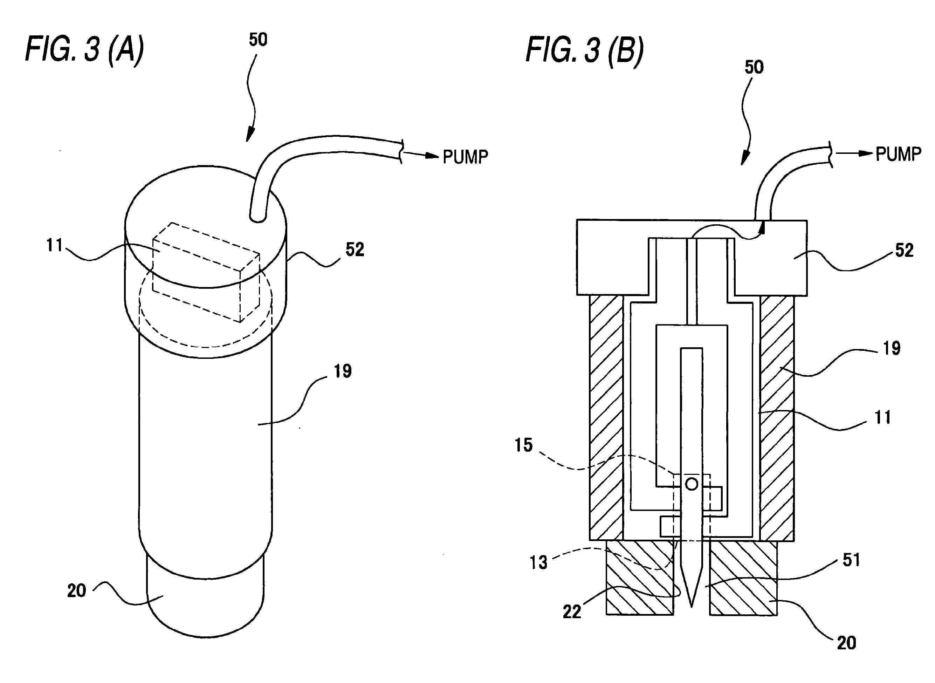

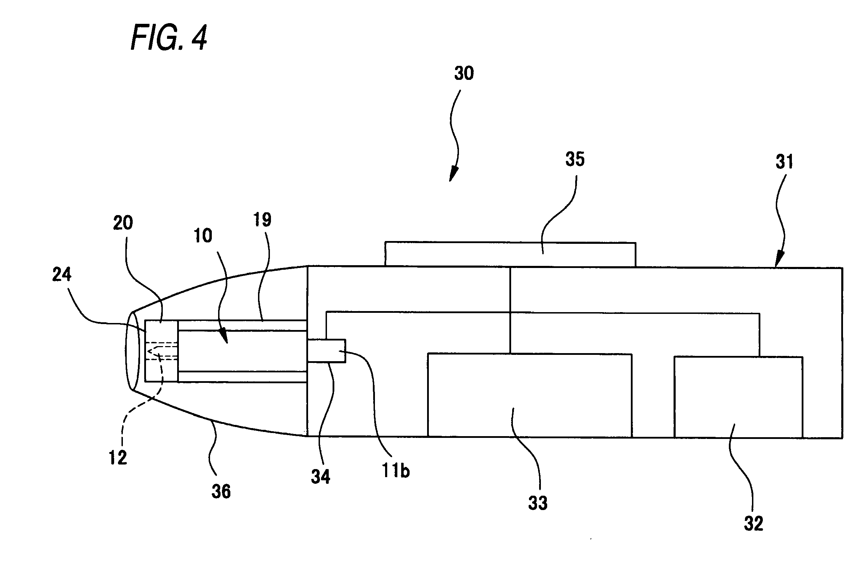

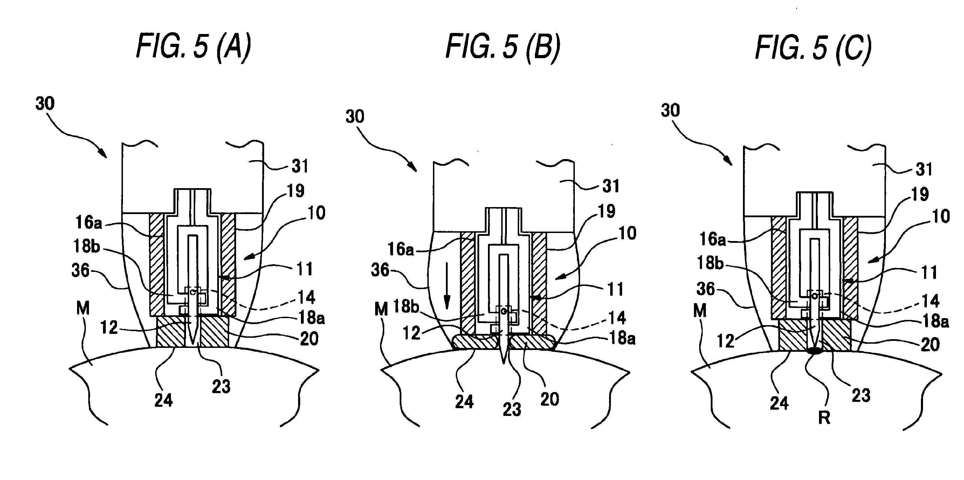

Fig. 1A is a sectional view of an A-A position in Fig. 1B showing a first embodiment according to a biosensor cartridge of the present invention, Fig. 1B is a sectional view of a B-B position in Fig. 1A showing the first embodiment according to the biosensor cartridge of the present invention, Fig. 2 is an exploded perspective view showing an example of a cover member, Fig. 3A is a perspective view showing an example of a suction unit used for a sample collecting method according to the present invention, Fig. 3B is a sectional view, Fig. 4 is a configuration diagram showing a first embodiment according to a biosensor device of the present invention, and Figs. 5A to 5C are explanatory views showing collecting operation of blood, which is a sample, using the biosensor device of Fig. 4.

As shown in Figs. 1A and 1B, biosensor cartridge 10 according to the first embodiment of the present invention has biosensor chip body 11, and puncturing tool 12 which is fixed to tip portion 11a of biosensor chip body 11, and from which tip 12a protrudes. By pushing the tip portion against the subject M, elastic body 20 which forms enclosed space 23 so as to contain sample collection opening 13 provided at tip 11a of biosensor chip body 11, and a puncture hole formed in the subject M by puncturing tool 12 is provided at tip 11a of biosensor chip body 11, and cover member 19 is provided around biosensor chip body 11.
In the present invention, it is desirable that puncturing tool 12 names a needle, a lancet needle, a cannula, etc. generically and is made of a biodegradable material.

Biosensor chip body 11 has two substrates 16a and 16b which face each other, and spacer layer 17 which is sandwiched between two substrates 16a and 16b. The surface of at least one substrate 16a of two substrates 16a and 16b on the side of spacer layer 17 is provided with detecting electrodes 18a and 18b, and tip portions (lower ends in Fig. 1A) of the detecting electrodes are bent in an L shape in directions in which they face each other, and holds a predetermined interval therebetween. Hollow reaction portion 15 is formed by two substrates 16a and 16b and spacer layer 17 from tip 11a of biosensor chip body 11 to a portion where the two detecting electrodes 18a and 18b face each other. The tip (lower end in Fig. 1) of hollow reaction portion 15 is provided with sample collection opening 13 through which blood R (refer to Fig. 5C) as a sample collected by puncturing the sample M (refer to Fig. 5) with puncturing tool 12 is introduced into hollow reaction portion 15.

That is, in hollow reaction portion 15, both upper and lower surfaces are formed by substrates 16a and 16b and detecting electrodes 18a and 18b, and a rectangular space is formed with spacer layer 17 cut away in a predetermined shape as a side wall. For this reason, in hollow reaction portion 15, detecting electrodes 18a and 18b are exposed, and reagent 14 which immobilizes, for example, an enzyme and a mediator, and reacts with glucose in blood R to generate an electric current is provided immediately above or in the vicinity of detecting electrodes 18a and 18b in hollow reaction portion 15. Accordingly, hollow reaction portion 15 becomes a portion where blood R, such as blood collected from sample collection opening 13, biochemically reacts with reagent 14.

As reagent 14, enzymes and electron acceptors, such as glucose oxidase (GOD), glucose dehydrogenase (GDH), cholesterol oxidase, and uricase, are exemplified.
For example, in the case of a glucose biosensor chip which measures the amount of glucose in blood, this portion is formed with a glucose oxidase layer, a glucose oxidase electron acceptor (mediator) mixture layer, a glucose oxidase albumin mixture layer, or a glucose oxidase electron acceptor-albumin mixture layer. These layers may be formed using enzymes other than a glucose oxidase, for example, glucose dehydrogenase or the like. Additionally, a buffering agent, a hydrophilic polymer, etc. as additive agents may be included into the reagent.

As shown in Figs. 1 and 2, as elastic body 20 attached to tip 11a of biosensor chip body 11, for example, a cylindrical elastic body which has through hole 22 for forming enclosed space 23 in a central portion can be exemplified. Through hole 22 is larger than the external diameter of puncturing tool 12 so that puncturing tool 12 is inserted therethrough. Additionally, the thickness of elastic body 20 is set to a thickness such that even the tip of puncturing tool 12 can be surely covered.

In addition, the material for elastic body 20 is not particularly limited so long as it has elasticity. However, rubber, such as silicone, urethane, and acrylic rubber, rubber or sponge including a polymer simple substance or a copolymerized polymer, such as, ethylene, and styrene, polyolefine, such as polyethylene and polypropylene, polyester, such as polyethylene terephthalate and polybutylene terephthalate, and fluororesin, such as PFA that is a copolymer of polytetrafluoroethylene and perfluoro alkoxy ethylene, and polyfluoroethylene, etc. can be utilized. The rubber elastic body may be solid or hollow.

It is desirable that the tip face 21 that is the face of elastic body 20 which touches the subject M is made of a material having stickiness, such as silicone rubber and acrylic rubber, or the elastic body has gluing agent 24, or is coated with gluing agent 24. Thereby, the adhesion between elastic body 20 and the subject M can be improved, deviation from a puncturing position can be prevented, and enclosed space 23 can be surely formed. Additionally, it is desirable that the inner peripheral surface of through hole 22 is formed using a hydrophilic material, or at least the inner peripheral surface is subjected to hydrophilic treatment. Thereby, blood R to be collected can be easily passed through the through hole, and even a small amount of blood R can be surely collected.

As shown in Fig. 2, cover member 19 attached to the outside of biosensor chip body 11 has, for example, a cylindrical or prismatic cross-section. By adopting such a shape, cover member 19 can be easily created, and the strength of cover member 19 can be secured.

Slit 19a corresponding to the size of biosensor chip body 11 is provided in cover member 19 along its longitudinal direction (in a vertical direction in Fig. 2) so as to pass therethrough, so that biosensor chip body 11 can be pushed into and fixed to slit 19a. Thereby, since cover member 19 prevents from the axial center of puncturing tool 12 from deviating when puncturing is performed using biosensor cartridge 10 having flexibility, puncturing can be performed accurately in a desired position. Additionally, tip face 11a of biosensor chip body 11 and the tip face 19b of cover member 19 are arranged on the same plane, and elastic body 20 is bonded to both tip faces 11a and 19b with adhesive 27. Accordingly, the area of a surface which bonds elastic body 20 becomes large, so that elastic body 20 can be firmly attached. Additionally, since elastic body 20 can be fixed without forming a gap between elastic body 20 and biosensor chip body 11, blood R collected from a junction between elastic body 20 and biosensor chip body 11 can be prevented from leaking. In addition, it is necessary to take care so that adhesive 27 does not protrude into the portion which becomes a flow passage for blood R, and the inside of biosensor chip body 11.

As shown by two-point chain lines in Fig. 1, it is desirable that connector 41 for attaching biosensor cartridge 10 to driving unit 40, for example, like a lancet which performs puncture using biosensor cartridge 10, is attached to driving unit 40 via spring 42. Thereby, puncturing can be performed by the force of spring 42, and a burden on the subject M can be reduced by performing puncture in a short time. Additionally, when biosensor cartridge 10 is attached to connector 41, the cartridge is attached along with cover member 19, whereby attachment area becomes large. Thus, biosensor cartridge 10 can be firmly attached to driving unit 40.

Additionally, as shown in Fig. 3, blood R can be sucked and collected from sample collection opening 13 by using suction unit 50 connected to biosensor cartridge 10. That is, sealed passage 51 which extends to hollow reaction portion 15 through sample collection opening 13 from through hole 22 of elastic body 20 is formed, and the inside of sealed passage 51 is sucked. For this reason, connecting member 52 is attached to an upper end (rear end 11b) of biosensor cartridge 10, on which cover member 19 is mounted, in a sealed state, and the inside of connecting member 52 is sucked by a pump, etc. Thereby, since puncturing is performed using puncturing tool 12 of biosensor cartridge 10, and blood R is collected from sample collection opening 13 provided at tip 11a of biosensor cartridge 10, even a small amount of blood R is reliably collected without aligning sample collection opening 13 with a puncturing position.

Next, the biosensor device in the first embodiment of the present invention will be described. The configuration of biosensor device 30 using the above-described biosensor cartridge 10 is shown in Fig. 4.
As shown in Fig. 4, biosensor device 30 has aforementioned biosensor cartridge 10, and measuring instrument 31 and protective cap 36 which are connected to detecting electrodes 18a and 18b of biosensor cartridge 10, and obtains information on the collected blood R. In addition, the configuration of biosensor cartridge 10 is as described above. Thus, the same parts common to those of aforementioned biosensor cartridge 10 are denoted by the same reference numerals, and the description thereof is omitted.

Measuring instrument 31 includes power source 32, control device 33, terminal insertion portion 34, and display unit 35, and these are connected to each other. Rear end 11b of biosensor chip 11 in biosensor cartridge 10 is inserted into and fixed to terminal insertion portion 34, and detecting electrodes 18a and 18b exposed to rear end 11b of biosensor chip body 11 are electrically connected to each other. Biosensor device 30 is small-sized, and is, for example, a handicap type which can be gripped by a subject's single hand. Additionally, although not shown, it is also possible to provide driving unit 40 which drives biosensor cartridge 10, and suction unit 50 for sucking blood R inside biosensor device 30.

Next, a method of using the biosensor device will be described with reference to Figs. 5A to 5C, taking a case where the blood sugar level is measured using biosensor device 30 as an example.
First, as shown in Fig. 4, rear end 11b of main body 11 of biosensor cartridge 10 is inserted into, fixed to, and electrically connected to terminal insertion portion 34 of measuring instrument 31. Power source 32 of biosensor device 30 is switched on, and whether or not the power source is normally started is confirmed. As shown in Fig. 5A, biosensor device 30 is held, protective cap 36 is pushed against the subject M to congest a puncturing place, and elastic body 20 attached to tip 11a of biosensor cartridge 10 is brought into contact with a blood collecting place of the subject M. Since the tip face of elastic body 20 is coated with gluing agent 24, or elastic body 20 has stickiness, positional deviation can be prevented in the subsequent operation.

Next, as shown in Fig. 5B, biosensor cartridge 10 is pushed against the subject M such that a pushing force acts mainly on cover member 19. Thereby, elastic body 20 is crushed, and puncturing tool 12 protrudes from the tip of elastic body 20 to puncture the subject M. Additionally, when elastic body 20 is pushed against the subject M at this time, the periphery of the portion corresponding to through hole 22 is hold down, whereby the portion corresponding to through hole 22 is congested to make blood R flow out. In addition, in a case where aforementioned driving unit 40 is provided, puncturing can be performed in a short time by the restoring force of spring 42. Additionally, since the pushing force acts mainly via cover member 19, deformation of thin and flexible biosensor cartridge 10 can be prevented, and deviation of the axial center of puncturing tool 12 can be prevented.

As shown in Fig. 5C, when the force which pushes biosensor cartridge 10 is weakened, or spring 42 of driving unit 40 contracts, elastic body 20 returns to its original state (state of Fig. 5A) by a restoring force. Thus, puncturing tool 12 slips out of the subject M. Since the inside of enclosed space 23 including a puncture hole becomes negative pressure at this time, the blood R flows out of the puncture hole easily. Additionally, since the inner peripheral surface of through hole 22 which forms enclosed space 23 is subjected to hydrophilic treatment, blood R is collected from sample collection opening 13 along the inner peripheral surface of through hole 22 by the surface tension and capillary phenomenon thereof. In addition, in a case where aforementioned suction unit 50 is provided, enclosed space 23 can be further brought into negative pressure, and even a minute amount of blood R can be reliably collected.

The collected blood R is introduced into hollow reaction portion 15. At this time, since sample collection opening 13 is located within enclosed space 23 along with a puncture hole formed by puncturing tool 12, the blood R can be easily and reliably collected without moving biosensor cartridge 10. For this reason, since the biosensor device can be used even in the subject M whose eyesight is weakened, and measurement can be performed with a small amount of blood, a burden on the subject M when blood is collected can be reduced. Additionally, since enclosed space 23 is shut off from external air, coagulation of the blood R is delayed, thereby facilitating collecting.

If a predetermined amount of blood has been collected, biosensor device 30 is separated from the subject M, and waits for a test result to appear on display unit 35. The blood R introduced into hollow reaction portion 15 reacts with reagent 14, and data of a current value or charge value (charge amount) measured by detecting electrodes 18a and 18b is sent to control device 33. A calibration curve data table is stored in control device 33, and calculation of blood sugar level is executed on the basis of the measured current value (charge value). If the calculation is ended, a test result is displayed on display unit 35, for example, the blood sugar level can be expressed as a numerical value. Finally, although biosensor cartridge 10 is detached from measuring instrument 31, elastic body 20 returns to almost its original height at this time. Thus, puncturing tool 12 is in a state where it does not protrude from tip 11a of biosensor chip body 11. Thereby, a user is not damaged by puncturing tool 12, but the used biosensor cartridge 10 can be properly processed.

In addition, if the blood collecting burden of a subject is taken into consideration, it is preferable that the volume of hollow reaction portion 15 be equal to or less than 1 µL (microliter), especially, equal to or less than 300 nL (nanoliter). If hollow reaction portion 15 is minute in this way, a, sufficient amount of blood of the subject can be collected even if the diameter of puncturing tool 12 is small. Preferably, the diameter is equal to or less than 1000 pm.

As described above, according to the aforementioned biosensor cartridge 10 and biosensor device 30, when tip portion 11a of biosensor chip body 11 is pushed against the subject M, elastic body 20 provided at tip portion 11a of biosensor chip body 11 is compressed, and puncturing tool 12 for puncturing protrudes. Thus, the subject M can be punctured. Additionally, if a pushing force is weakened, puncturing tool 12 is extracted from the subject M by the restoring force of elastic body 20, and the blood R flows out of the puncture hole. In this case, since the puncture hole, and sample collection opening 13 provided at tip 11a of biosensor chip body 11 are contained in enclosed space 23 formed by elastic body 20, even a small amount of blood R is also easily collected by sample collection opening 13. Additionally, cover member 19 is provided around biosensor chip body 11. Thus, when puncturing is performed by thin plate-shaped biosensor cartridge 10, biosensor chip body 11 can be prevented from deforming, and puncturing can be accurately performed in a desired puncturing position. In addition, puncturing tool 12 is prevented from protruding from the tip face of elastic body 20 before use, so that protection of puncturing tool 12 and protection of a user can be achieved. Additionally, even when being discarded after use, puncturing tool 12 is prevented from protruding from the tip face of elastic body 20, so that the tool can be safely and properly disposed. Moreover, since cover member 19 is provided even when biosensor cartridge 10 is mounted on driving unit 40, contact area is large, and the mounting is easy. Additionally, since the area of contact with elastic body 20 becomes large, setting to the elastic body is easy, and the problem that the elastic body is separated from biosensor cartridge 10 can be solved.

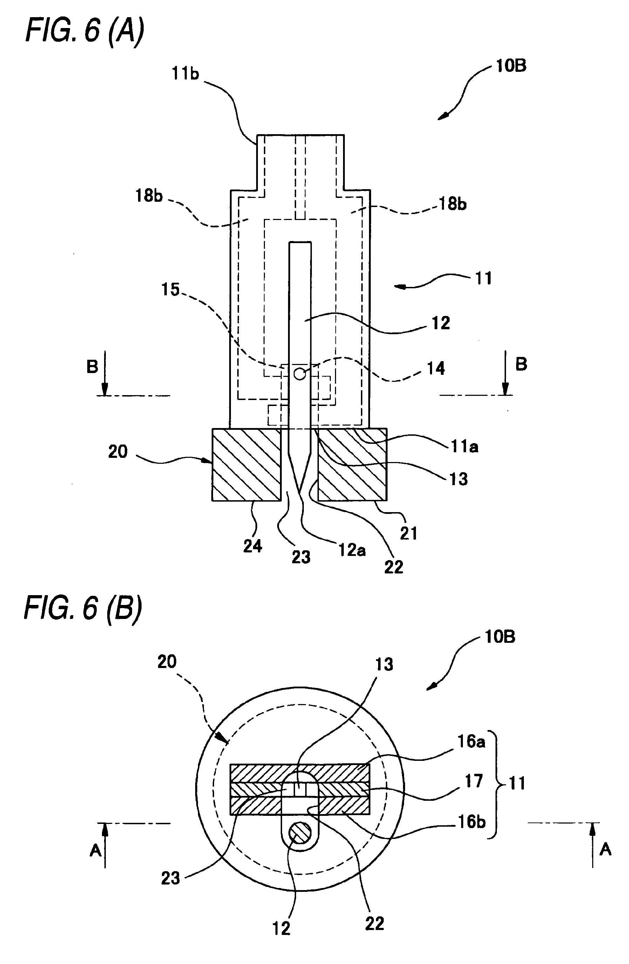

In addition, the biosensor chip of the present invention is not limited to the aforementioned first embodiment, and proper modifications, improvements, etc. can be made.
For example, although the case where puncturing tool 12 is provided inside biosensor chip body 11, i.e., is provided in spacer layer 17 sandwiched between both substrates 16a and 16b has been illustrated in the aforementioned first embodiment, biosensor cartridge 10 of the present invention is not limited thereto. For example, as shown in Figs. 6A and 6B, puncturing tool 12 can also be provided along the outside surface of one substrate 16a. In the case of a biosensor cartridge 10B, the thin biosensor cartridge 10 can be formed by reducing the thickness of biosensor chip body 11. In this regard, since puncturing tool 12 and sample collection opening 13 are somewhat separated from each other, it is desirable to make the cross-sectional shape of through hole 22 oval or the like, thereby making as small as possible a gap formed between the outer peripheral surface of puncturing tool 12 and the inner peripheral surface of through hole 22 of elastic body 20. In addition, in Fig. 6, parts common to those of biosensor cartridge 10 which have been already described are denoted by the same reference numerals, and overlapping description is omitted.
Additionally, it is also possible to make biosensor cartridge 10 or cover member 19, and elastic body 20 fit to each to by a convexo-concave structure.
Moreover, a motor other than a spring can be used as the driving unit.

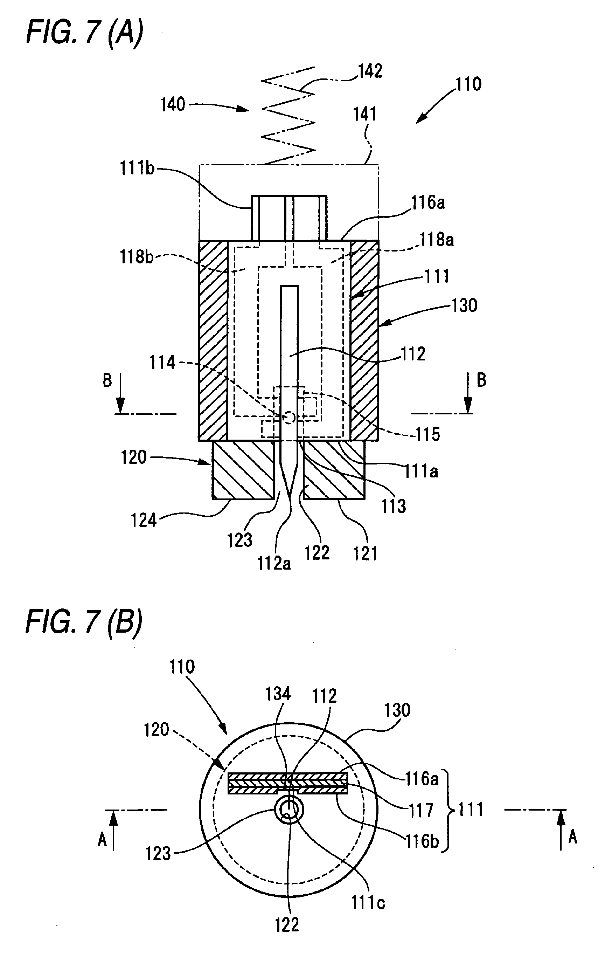

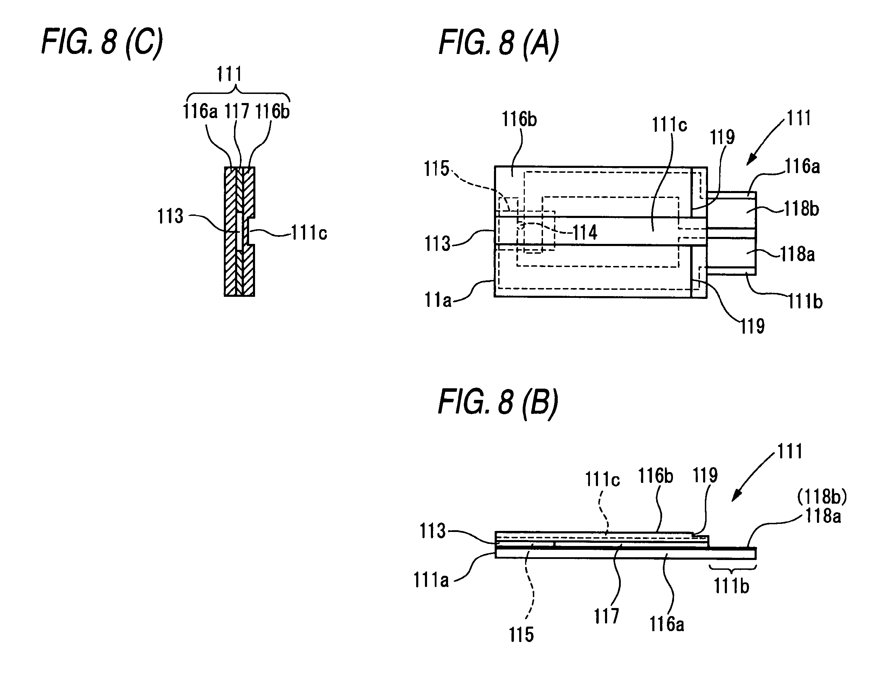

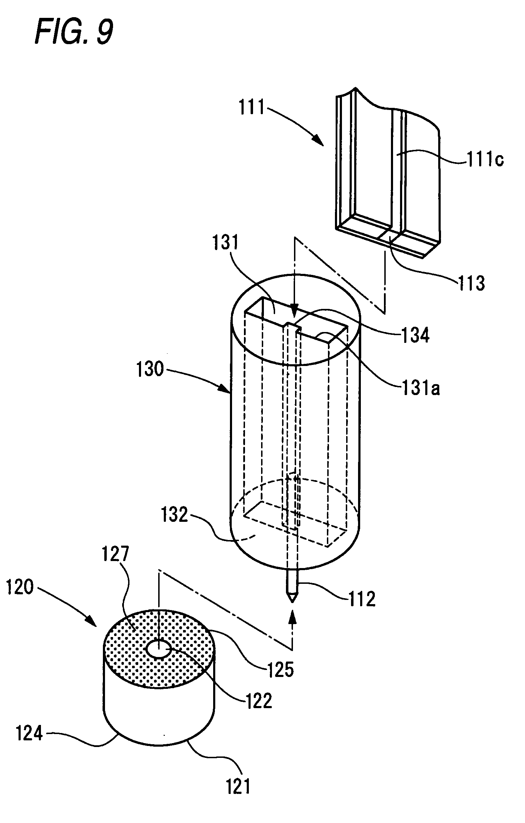

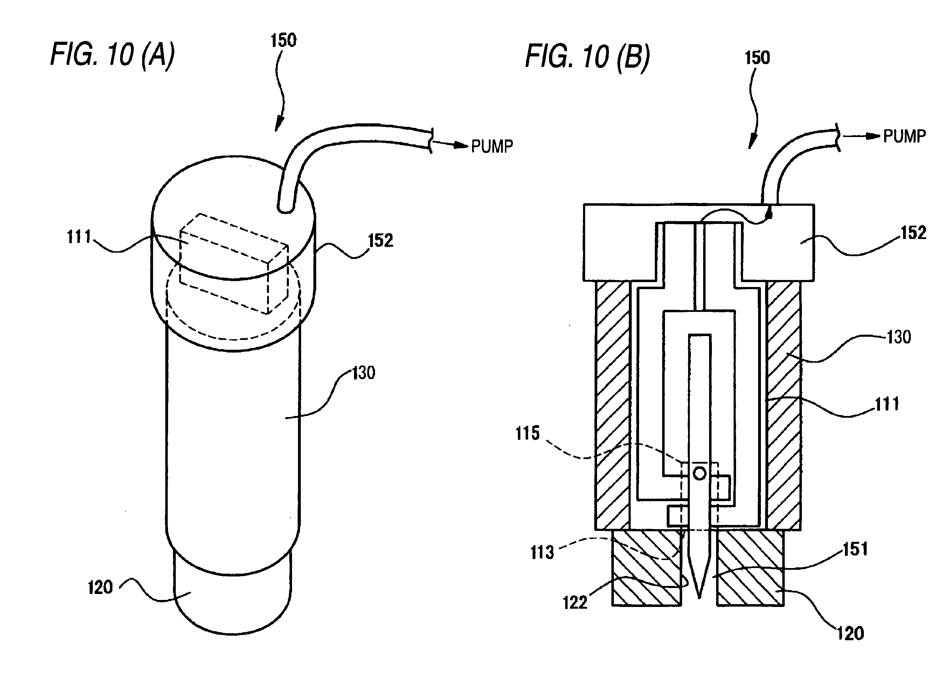

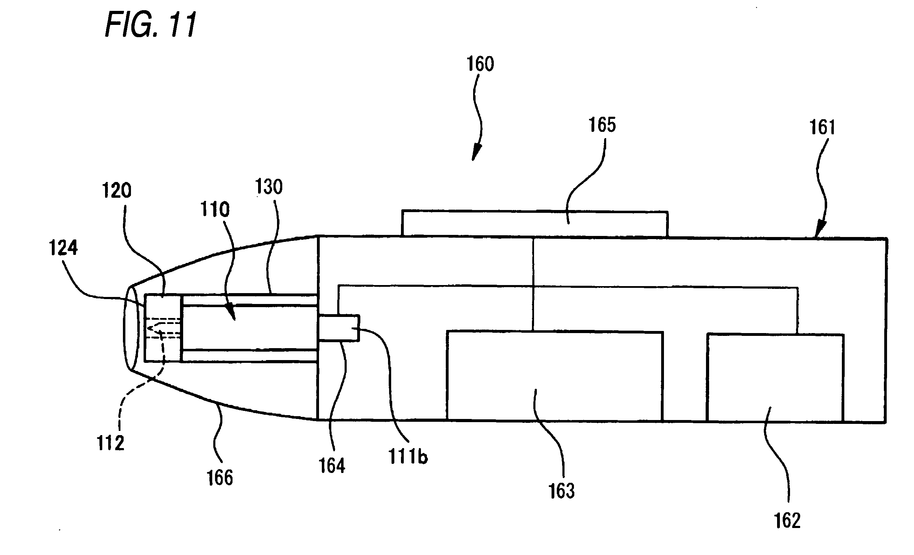

Hereinafter, a second embodiment according to the present invention will be described in detail with reference to the drawings. Fig. 7A is a sectional view of an A-A position in Fig. 7B showing a second embodiment according to the biosensor cartridge of the present invention, Fig. 7B is a sectional view of a B-B position in Fig. 7A showing the second embodiment according to the biosensor cartridge of the present invention, Fig. 8A is a plan view of a biosensor chip, Fig. 8B is a side view of the biosensor chip, Fig. 8C is a front end view of the biosensor chip, Fig. 9 is an exploded perspective view showing a method of fixing the cover member and biosensor chip, Fig. 10A is a perspective view showing an example of the suction unit, Fig. 10B is a sectional view, and Fig. 11 is a configuration diagram showing an example of a biosensor device in a second embodiment of the present invention.

As shown in Figs. 7A and 7B, biosensor cartridge 110 according to the second embodiment of the present invention has biosensor chip 111, and puncturing tool 112 which is fixed to tip portion 111a of biosensor chip 111, and from which a tip 112a protrudes. By pushing the tip portion against a subject, an elastic body 120 which forms an enclosed space 123 so as to contain sample collection opening 113 provided at the tip 111a of biosensor chip 111, and a puncture hole formed in the subject by puncturing tool 112 is provided at the tip 111a of biosensor chip 111, and cover member 130 is provided around biosensor chip 111.
In addition, in the present invention, it is desirable that puncturing tool 112 names a needle, a lancet needle, a cannula, etc. generically and is made of a biodegradable material.

As shown in Figs. 7 and 8, biosensor chip 111 has two substrates 116a and 116b which face each other, and spacer layer 117 which is sandwiched between the two substrates 116a and 116b. Recess 111c is formed in the surface of the substrate 116b of the two substrates 116a and 116b nearer to puncturing tool 112 along the longitudinal direction. The surface of at least one substrate 116a of the two substrates 116a and 116b on the side of spacer layer 117 is provided with detecting electrodes 118a and 118b, and tip portions (lower ends in Fig. 7A) of the detecting electrodes are bent in an L shape in directions in which they face each other, and holds a predetermined interval therebetween. A hollow reaction portion 115 is formed by the two substrates 116a and 116b and spacer layer 117 from the tip 111a of biosensor chip 111 to a portion where the two detecting electrodes 118a and 118b face each other. The tip (lower end in Fig. 7) of the hollow reaction portion 115 is provided with sample collection opening 113 through which blood as a sample collected by puncturing a subject with puncturing tool 112 is introduced into the hollow reaction portion 115.

That is, in the hollow reaction portion 115, both upper and upper surfaces are formed by the substrates 116a and 116b and the detecting electrodes 118a and 118b, and a rectangular space is formed with spacer layer 117 cut away in a predetermined shape as a side wall. For this reason, in the hollow reaction portion 115, the detecting electrodes 118a and 118b are exposed, and a reagent 114 which immobilizes, for example, an enzyme and a mediator, and reacts with glucose in blood to generate an electric current is provided immediately above or in the vicinity of the detecting electrodes 118a and 118b in the hollow reaction portion 115. Accordingly, the hollow reaction portion 115 becomes a portion where a sample, such as blood collected from sample collection opening 113, biochemically reacts with the reagent 114. The reagent 114 includes, for example, glucose oxidase (GOD).

As shown in Figs. 7 and 9, as elastic body 120 attached to the tip 111a of biosensor chip 111, for example, a cylindrical elastic body which has a through hole 122 for forming the enclosed space 123 in a central portion can be exemplified. The through hole 122 is larger than the external diameter of puncturing tool 112 so that puncturing tool 112 is inserted therethrough. Additionally, the thickness of elastic body 120 is set to a thickness such that even the tip of puncturing tool 112 can be surely covered. In addition, the material for elastic body 120 is not particularly limited so long as it has elasticity. However, rubber, such as silicone, urethane, and acrylic rubber, rubber or sponge including a polymer simple substance or a copolymerized polymer, such as, ethylene, and styrene, polyolefine, such as polyethylene and polypropylene, polyester, such as polyethylene terephthalate and polybutylene terephthalate, and fluororesin, such as PFA that is a copolymer of polytetrafluoroethylene and perfluoro alkoxy ethylene, and polyfluoroethylene, etc. can be utilized. In addition, elastic body 120 may be solid or hollow.

It is desirable that the tip face 121 that is the face of elastic body 120 which touches a subject is made of a material having stickiness, such as silicone rubber and acrylic rubber, or elastic body 120 is coated with the gluing agent 124. Thereby, the adhesion between elastic body 120 and the subject can be improved, deviation from a puncturing position can be prevented, and the enclosed space 123 can be surely formed. Additionally, it is desirable that the inner peripheral surface of the through hole 122 is formed using a hydrophilic material, or at least the inner peripheral surface is subjected to hydrophilic treatment. Thereby, blood to be collected can be easily passed through the through hole, and even a small amount of blood can be surely collected, and guided to the hollow reaction portion.

As shown in Fig. 9, cover member 130 attached to the outside of biosensor chip 111 has, for example, a cylindrical or prismatic cross-section. By adopting such a shape, cover member 130 can be easily created, and the strength of cover member 130 can be secured. A fitting hole 131 corresponding to the size of biosensor chip 111 is provided in cover member 130 along its longitudinal direction (in a vertical direction in Fig. 9) so as to pass therethrough, and the inner surface of fitting hole 131 is provided with protrusion 134 which fits to recess 111c of biosensor chip 111. Additionally, puncturing tool 112 which protrudes from the tip face 132 of the cover 130 is provided integrally in the vicinity of fitting hole 131 inside cover member 130.

Accordingly, cover member 130 can be fixed to the outside of biosensor chip 111 by fitting biosensor chip 111 into fitting hole 131 such that protrusion 134 is fitted to he recess 111c of biosensor chip 111. Thereby, since cover member 130 prevents from the axial center of puncturing tool 112 from deviating when puncturing is performed using biosensor cartridge 110 having flexibility, puncturing can be performed accurately in a desired position. Additionally, tip face 111a of biosensor chip 111 is exposed to the tip of fitting hole 131, and tip face 111a of biosensor chip 111 and the tip face 132 of cover member 130 are arranged on the same plane, and elastic body 120 is bonded to both the tip faces 111a and 119b with an adhesive 127. Accordingly, the area of a surface which bonds elastic body 120 becomes large, so that elastic body 120 can be firmly attached. Additionally, since elastic body 120 can be fixed without forming a gap between elastic body 120 and biosensor chip 111, blood collected from a junction between elastic body 120 and biosensor chip 111 can be prevented from leaking. In addition, it is necessary to take care so that the adhesive 127 does not protrude into the portion which becomes a flow passage for the blood, and the inside of biosensor chip 111.

As shown by two-point chain lines in Fig. 7, it is desirable that a connector 141 for attaching biosensor cartridge 110 to driving unit 140, for example, like a lancet which performs puncture using biosensor cartridge 110, is attached to driving unit 140 via a spring 142. Thereby, puncturing can be performed by the force of the spring 142, and a burden on the subject can be reduced by performing puncture in a short time. Additionally, when biosensor cartridge 110 is attached to the connector 141, the cartridge is attached along with cover member 130, whereby attachment area becomes large. Thus, biosensor cartridge 110 can be firmly attached to driving unit 140.

Additionally, as shown in Fig. 10, blood can be sucked and collected from sample collection opening 113 by using suction unit 150 connected to biosensor cartridge 110. That is, a sealed passage 151 which extends to the hollow reaction portion 115 through sample collection opening 113 from the through hole 122 of elastic body 120 is formed, and the inside of the sealed passage 151 is sucked. For this reason, a connecting member 152 is attached to an upper end (rear end 111b) of biosensor cartridge 110, on which cover member 130 is mounted, in a sealed state, and the inside of the connecting member 152 is sucked by a pump, etc. Thereby, since puncturing is performed using puncturing tool 112 of biosensor cartridge 110, and blood is collected from sample collection opening 113 provided at the tip 111a of biosensor cartridge 110, even a small amount of blood is reliably collected without aligning sample collection opening 113 with a puncturing position.

Next, the biosensor device in the second embodiment of the present invention will be described. The configuration of biosensor device 160 using the above-described biosensor cartridge 110 is shown in Fig. 11.
As shown in Fig. 11, biosensor device 160 has aforementioned biosensor cartridge 110, and measuring instrument 161 and protective cap 166 which are connected to the detecting electrodes 118a and 118b of biosensor cartridge 110, and obtains information on the collected blood. In addition, the configuration of biosensor cartridge 110 is as described above. Thus, the same parts common to those of the aforementioned biosensor cartridge 110 are denoted by the same reference numerals, and the description thereof is omitted.

Measuring instrument 161 includes power source 162, control device 163, terminal insertion portion 164, and display unit 165, and these are connected to each other. Rear end 111b of biosensor chip 111 in biosensor cartridge 110 is inserted into and fixed to terminal insertion portion 164, and the detecting electrodes 118a and 118b exposed to rear end 111b of biosensor chip body 111 are electrically connected to each other. Biosensor device 160 is small-sized, and is, for example, a handicap type which can be gripped by a subject's single hand. Additionally, although not shown herein, it is also possible to provide driving unit 140 which drives biosensor cartridge 110, and suction unit 150 for sucking blood inside biosensor device 160.

Next, a method of manufacturing the biosensor cartridge in the second embodiment of the present invention will be described.
As shown in Fig. 9, the method of manufacturing a biosensor cartridge is a method of manufacturing biosensor cartridge 110 having biosensor chip 111 which has sample collection opening 113 at tip face 111a thereof, and puncturing tool 112 which is fixed to tip portion 111a of biosensor chip body 111, and from which tip 112a protrudes. Also, the method includes a step of making fitting hole 131 as a first fitting portion provided in cover member 130, which has puncturing tool 112 integrally, fit to biosensor chip body 111 as a second fitting portion.

That is, as shown in Figs. 8 and 9, in biosensor chip 111 formed by a laminated structure of upper and lower substrates 116a and 116b and spacer layer 117, recess 111c is formed in any one of the upper and lower surfaces of the biosensor chip in its longitudinal direction. Additionally, as shown in Fig. 9, protrusion 134 which fits to recess 111c of biosensor chip 111 is provided in inner surface 131a of fitting hole 131 of cover member 130 along the insertion direction of biosensor chip 111. Also, when biosensor chip 111 is inserted into cover member 130, protrusion 134 and recess 111c are made to fit to each other. Thereafter, elastic body 120 is attached to the tip faces of biosensor cartridge 110 and cover member 130 if needed.

As described above, according to the method of manufacturing a biosensor cartridge described above, protrusion 134 of cover member 130, and recess 111c of biosensor chip 111 are made to fit to each other, and thereby integrated. Thus, assembling is easy, integration can be firmly achieved, and puncturing tool 112 can be easily attached to biosensor chip 111. Additionally, since protrusion 134 is provided in cover member 130 in which puncturing tool 112 is integrally provided, puncturing tool 112 can be provided close to the surface which contacts biosensor chip 111, and puncturing tool 112 and sample collection opening 113 of biosensor chip 111 can be provided close to each other. Thereby, few samples can be reliably collected and measured, and a burden on a user can be reduced.

In addition, the case where fitting hole 131 in which protrusion 134 which protrude inward is provided is used as the first fitting portion, and biosensor chip body 111 in which recess 111c is provided is used as the second fitting portion has been described in the aforementioned second embodiment. On the contrary, however, the recess can be provided in fitting hole 131, and be used as the first fitting portion, and the protrusion can be provided in biosensor chip 111, and be used as a second fitting portion. Moreover, fitting hole 131 in which a protrusion and a recess are not provided can be used as the first fitting portion, and biosensor chip 111 in which a recess and a protrusion are not provided can be used as the second fitting portion.

### Working Example 1

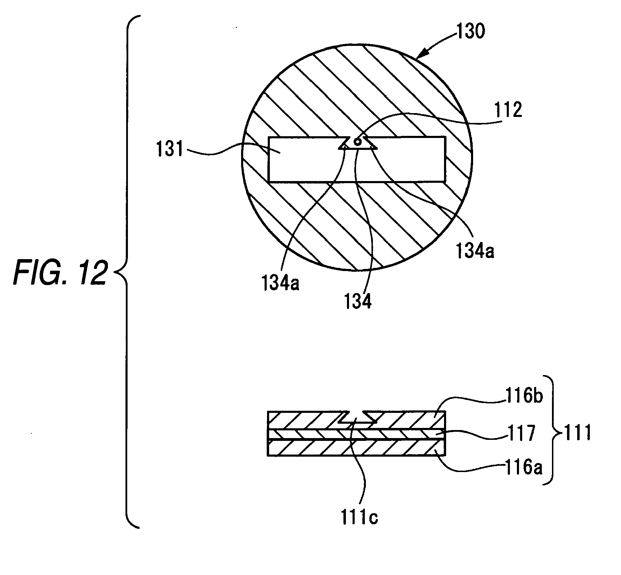

Working Example 1 is shown in Fig. 12. In addition, all parts common to the aforementioned parts are denoted by the same reference numerals, and overlapping description is omitted.
As shown in Fig. 12, biosensor chip 111 and puncturing tool 112 are fixed by inserting biosensor chip 111 into fitting hole 131 of cover member 130 while protrusion 134 of cover member 130 is made to fit to recess 111c of biosensor chip 111.
That is, for example, the tip portion of protrusion 134 inside fitting hole 131 of cover member 130 is made wider than the base end thereof, thereby providing swelling portion 134a, and the inside of recess 111c of biosensor chip 111 is widely provided so as to correspond to protrusion 134 of cover member 130.

Thereby, cover member 130 and biosensor chip 111 can be easily fixed only by inserting and fitting protrusion 134 into recess 111c. Additionally, puncturing tool 112 provided in cover member 130 can be easily attached to biosensor chip 111. In addition, when puncturing tool 112 is attached in the vicinity of or inside protrusion 134, the distance between puncturing tool 112 and sample collection opening 113 can be brought closer to each other. Thus, few samples can be reliably collected and measured, and a burden on a user can be reduced. Additionally, it is desirable that puncturing tool 112 is provided in protrusion 134 of cover member 130 to bring the puncturing position by puncturing tool 112 close to sample collection opening 113, so that a sample can be reliably collected.

### Working Example 2

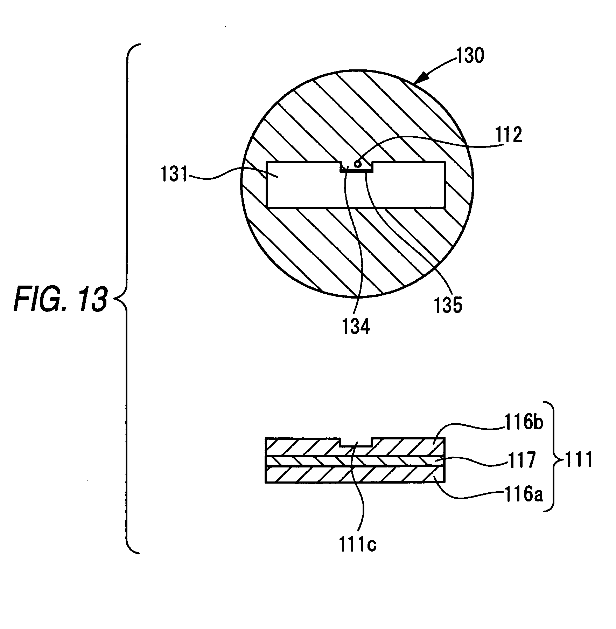

Working Example 2 is shown in Fig. 13. In addition, all parts common to the aforementioned parts are denoted by the same reference numerals, and overlapping description is omitted.
As shown in Fig. 13, biosensor chip 111 and puncturing tool 112 are fixed by inserting biosensor chip 111 into fitting hole 131 of cover member 130 while protrusion 134 of cover member 130 is made to fit to recess 111c of biosensor chip 111, and joining biosensor chip 111 and cover member 130 with adhesive 135. Although adhesive 135 can be applied to, for example, the tip face of protrusion 134 of cover member 130, the adhesive can also be applied to the inner surface the other fitting hole 131.

Thereby, cover member 130 and biosensor chip 111 can be easily fixed, and puncturing tool 112 provided in cover member 130 can be easily attached to biosensor chip 111. Additionally, it is desirable that puncturing tool 112 is provided in protrusion 134 of cover member 130 to bring the puncturing position by puncturing tool 112 close to sample collection opening 113, so that a sample can be reliably collected.

### Working Example 3

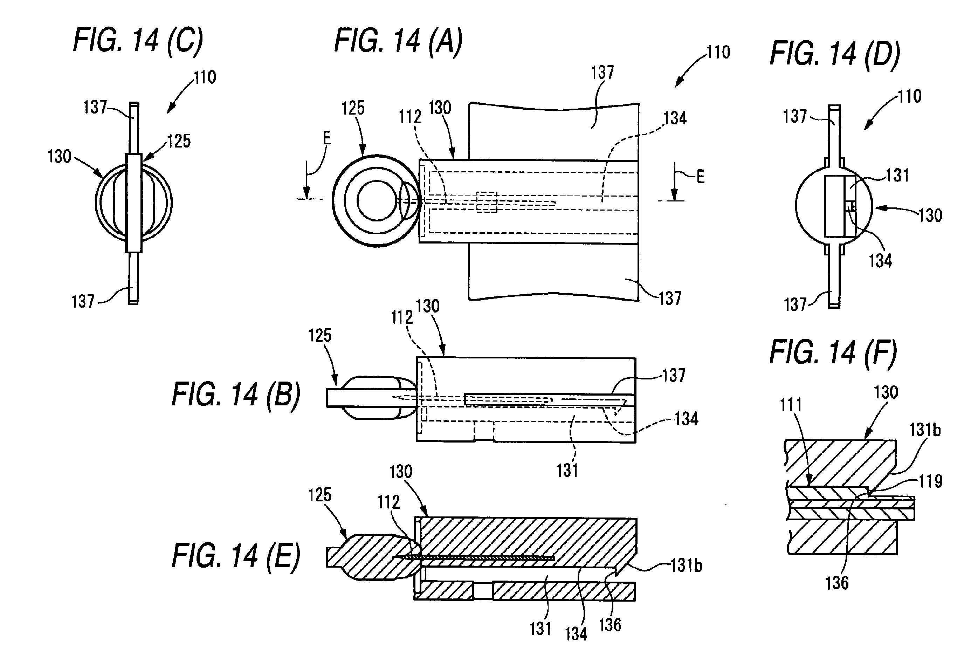

Working Example 3 is shown in Figs. 14A to 14F. In addition, all parts common to the aforementioned parts are denoted by the same reference numerals, and overlapping description is omitted.
As shown in Fig. 14, cover member 130 includes locking claw 136 in the vicinity of the rear end thereof in the insertion direction, and biosensor chip 111 and puncturing tool 112 are fixed by locking stepped portion 119 (refer to Fig. 8) provided in biosensor chip 111. In addition, as shown in Fig. 14F, it is desirable that inclined surface 131b is provided at the rear end of fitting hole 131, and locking claw 136 is provided at the tip of inclined surface 131b, thereby making biosensor chip 111 easily inserted into fitting hole 131.

Additionally, in Working Example 3, elastic body 120 is not provided, but puncturing tool cover 125 is provided instead to prevent a user or the like from being wounded by puncturing tool 112 and protect the tip portion of puncturing tool 112. Additionally, a pair of right and left blade portions 137 is provided on the outside of cover member 130, so that a user can set biosensor cartridge 110 in biosensor device 160 with blade portions 137. In this case, biosensor cartridge 110 can be set without removing protective cap 166 by providing slits (not shown) where tip portions of blade portions 137 are slidable in a state where they protrude outward to protective cap 166 of biosensor device 160. In addition, the out edges of blade portions 37 are slightly dented at their central portions, so that a user easily grips them with his/her fingers.

Thereby, when biosensor chip 111 is inserted into cover member 130, stepped portion 119 provided at the rear end of biosensor chip 111 is locked by locking claw 136 provided at the rear end of cover member 130. Thus, biosensor chip 111 and cover member 130 can be reliably fixed.

In addition, the method of manufacturing a biosensor chip of the present invention is not limited to the aforementioned second embodiment, and proper modifications, improvements, etc. can be made.
For example, in the aforementioned second embodiment, the tip portion of biosensor chip cartridge 110 is provided with elastic body 120. However, the elastic body may not be provided. In this case, it is desirable to provide a protective cap for puncturing needle 112a.
Additionally, in the aforementioned second embodiment, puncturing tool 112 integrally provided in cover member 130 can also be provided inside protrusion 134. In this case, since puncturing tool 112 and sample collection opening 113 can be provided closer to each other, a sample can be reliably collected without aligning sample collection opening 113 with a puncturing place after being punctured by puncturing tool 112.

Additionally, in the aforementioned second embodiment mentioned, a cover member of a shape which completely contains biosensor chip 111 is illustrated as cover member 130. However, a partial columnar cover member to be attached only to one surface of biosensor chip 111 may be used. In this case, the surface of cover member 130 which touches biosensor chip 111 is provided with a protrusion or a recess serving as the first fitting portion, the surface of biosensor chip 111 which touches cover member 130 is provided with a recess or a protrusion, and the protrusion and the recess are made to fit to each other, so that cover member 130 and biosensor chip 111 can be integrated.

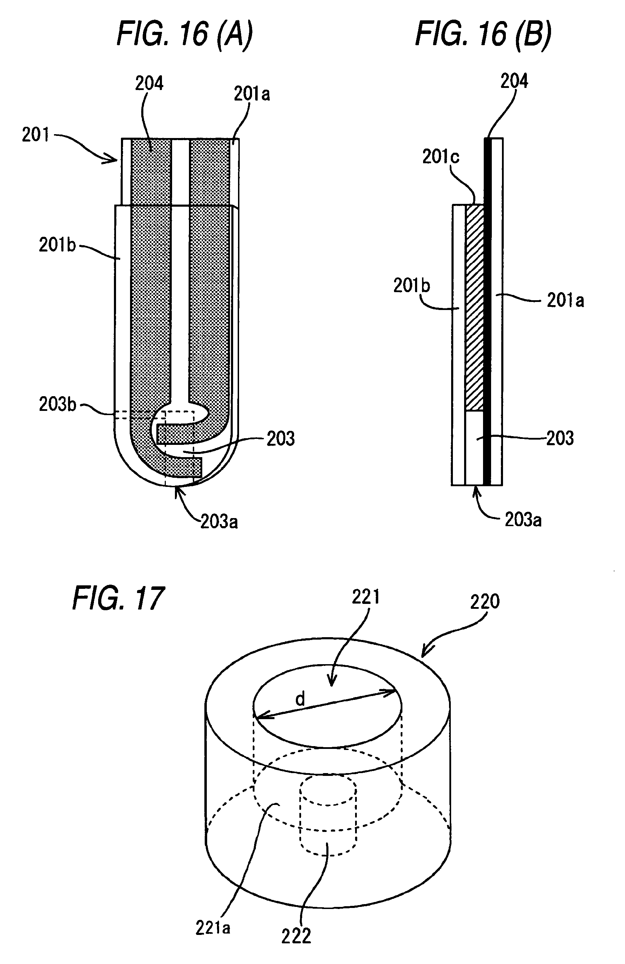

Next, one embodiment of a needle integral sensor of the present invention will described with reference to the drawings.

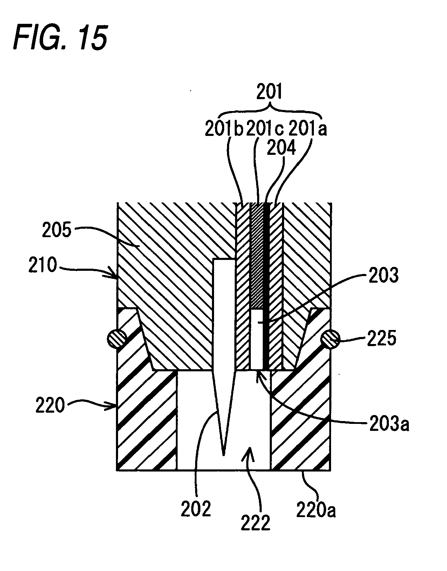

Fig. 15 is a sectional view of the needle integral sensor of this embodiment.
In the needle integral sensor of this embodiment, flow passage forming body 220 as shown in Fig. 17 is attached to needle integral sensor body 210 to which puncturing needle 202 is anchored so that the tip of the puncturing needle protrude onto one side of flat plate-like detecting portion 201 as shown in Fig. 16.

Flat plate-like detecting portion 201 is formed by sticking two electrically insulating substrates 201a and 201b together with an adhesive or the like. In Figs. 15 and 16, 201c represents an adhesive portion. A square portion to which the adhesive is not applied exists at the tip of detecting portion 201, and the square adhesive non-application portion forms reaction portion 203 which houses a liquid sample, such as blood. Additionally, the adhesive non-application portion exists from reaction portion 203 to a side end of insulating substrate 201a, and this adhesive non-application portion forms air hole 203b which allows reaction portion 203 and the outside of flat plate-like detecting portion 201 to communicate with each other. In Fig. 16, 203a represents an inlet of reaction portion 203.

A pair of detecting electrode patterns 204 is printed on the surface of insulating substrate 201a to which an adhesive is applied, and electrode patterns 204 are drawn so as to intersect reaction portion 203. Additionally, a reagent which reacts with a liquid sample, such as blood, is applied to reaction portion 203. Accordingly, the liquid sample contained in reaction portion 203 react with the reagent, so that a change in electric potential or current caused by a chemical change can be detected by the pair of electrodes 204. For example, when blood as the liquid sample is contained in reaction portion 203, an electrical change caused by reaction with the reagent is detected by electrodes 204, so that desired characteristics, such as blood sugar level, can be detected by a measurement unit.

Puncturing needle 202 is anchored by stacking a needle carrier 205 on the surface (often referred to as the "outside surface of an insulating substrate") of substrate 201b to which an adhesive is not applied. As the puncturing needle, a hollow needle used for a general syringe, a solid needle whose tip is sharp, a lancet needle, etc. can be used. Additionally, a mounting portion 206 for mounting a needle integral sensor body 210 on a measuring device is stacked on the outside surface of insulating substrate 201a, to which puncturing needle 202 is not attached, of two substrates 201a and 201b of flat plate-like detecting portion 201. Needle supporting substrate 205 and mounting portion 206 form a substantially columnar shape about puncturing needle 202 so that a tip portion to which puncturing needle 202 is attached can be inserted into recess 221 of flow passage forming body 220.

Flow passage forming body 220 is obtained by integrally forming a cylindrical attachment portion in which columnar recess 221 is recessed, and a cylindrical flow passage portion through which a through hole which becomes flow passage 222 passes. Flow passage forming body 220 is made of an elastic body or a viscoelastic body which can be deformed by a pressing for in a puncture direction, and be restored to its substantial original shape by release of pressure. Specifically, crude rubber; synthetic rubber, such as synthetic isoprene rubber, styrene rubber, nitrile rubber, chloroprene rubber, and acrylic rubber; rubber-like elastic body, such as silicone rubber and urethane rubber; thermoplastic elastomer, such as ethylene vinylacetate copolymer, and sponge, such as polystyrene foam, can be used.

The internal diameter d of recess 221 is larger than the external diameter of the tip portion of sensor body 210 inserted into recess 221, and the tip portion of sensor body 210 is fitted into recess 221 in a loosely fitting state. A through hole penetrating from bottom face 221a of recess 221 to subject abutting surface 220a of flow passage forming body 220 becomes flow passage 222 which leads to reaction portion inlet 203a from a puncturing position, and the tip of puncturing needle 202 is inserted into flow passage 222.

The outer periphery of a relevant portion of recess 221 of flow passage forming body 220 is fastened by C-shaped ring 225 that is a fastener, and thereby, flow passage forming body 220 which is fitted in a loosely fitting state is attached and fixed to needle integral sensor body 210.

The needle integral sensor having the configuration as described above can be manufactured first by inserting the tip of sensor body 210 into recess 221 of flow passage forming body 220, and then by fastening recess 221 from its outer periphery by C ring 225 that is a fastener. By fastening flow passage forming body 220 to sensor body 210 by C ring 225, flow passage forming body 220 which has been fitted by loose fitting can be attached and fixed to sensor body 210, and thereby, the position of flow passage forming body 220 can be prevented from deviating even at the time of pull-out of puncturing needle 202 by restoration of flow passage forming body 220 after puncture. Additionally, insertion operation of sensor body 210 in a loose fitting relation and attaching and fixing operation of flow passage forming body 220 by fastening by a fastener are simple, and deviation of attaching operation is little compared with a case where flow passage forming body and the sensor body are attached and fixed by tight fitting or a case where the flow passage forming body is anchored to the tip of the sensor body with an adhesive or the like. Particularly, in a case where the flow passage forming body is made of a soft viscoelastic material, handling is troublesome, and the operation of fitting sensor body 210 into flow passage forming body 220 in a tight fitting relation requires time and effort. In this regard, in the needle integral sensor of the present invention, only the fitting operation of the flow passage forming body in a loose fitting relation and fastening of C ring 225 are adopted. Therefore, attaching operation is simple, and productivity is excellent, regardless of the material for the flow passage forming body.

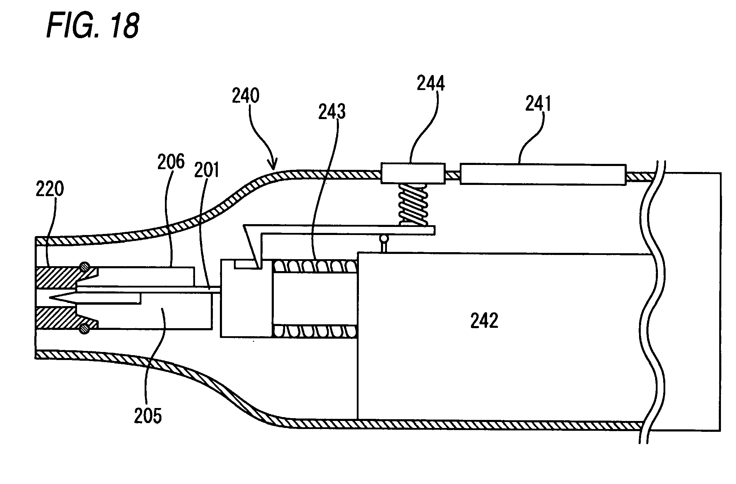

The needle integral sensor having the configuration as described above is mounted on measuring instrument 240 including display unit 241, measurement unit 242, spring 243 for puncturing, and spring-operated button 244, as shown in Fig. 18. At the time of mounting, spring 243 for puncturing is set in a compressed state by inserting mounting portion 206 of needle integral sensor body 210 into a set portion (not shown) of the measuring instrument. By pushing spring-operated button 244, spring 243 is released from the compressed state, so that needle integral sensor body 210 can be driven in the puncturing direction.

When subject abutting surface 220a of flow passage forming body 220 is pressed against the skin of a subject, for example, a finger in the compression state (spring-biased state) of the spring for puncturing, thereby operating a button in a direction in which the spring holding puncturing needle 202 extends, needle integral sensor body 210 is pushed out toward the skin. Along with this, a pressing force is generated in the puncturing direction in flow passage forming body 220 attached to the tip of needle integral sensor body 210, whereby flow passage forming body 220 is deformed so as to be compressed in the puncturing direction or expanded in a radial direction. Since puncturing needle 202 attached to the tip of needle integral sensor body 210 is pushed out toward a skin in a deformed state of flow passage forming body 220, puncturing needle 202 protrudes from subject abutting surface 220a of flow passage forming body 220, and the skin is punctured. Next, when the biasing by the spring is released, flow passage forming body 220 returns to its substantial original shape by its own restoring force, and along with this, puncturing needle 202 is pulled out of the skin and is housed in flow passage 222. The blood as a liquid sample discharged to the surface of the skin by the puncturing rises along the needle or along the inner wall surface of flow passage 222, and is further introduced into reaction portion 203 from reaction portion inlet 203a.

As described above, in the needle integral sensor of this embodiment, the blood can be contained in reaction portion 203 through flow passage 222 irrespective of whether the tip of puncturing needle 202 and reaction portion inlet 203a are in positions separated from each other. In addition, in order to effectively perform the rise of a liquid sample within flow passage 222, a ventilation passage which allows flow passage 222 and the outside of flow passage forming body 220 to communicate with each other may be provided in a proper position of the flow passage portion. In this case, it is preferable to form the ventilation passage as a curved passage or adjust the size of the ventilation passage so that a liquid sample which flows through the inside of flow passage 222 may not flow out of the ventilation passage, or to apply a surfactant to the inner wall surface of flow passage 222 or apply a surfactant in the vicinity of reaction portion inlet 203a and so that the surfactant rises preferentially inside flow passage 222 and is introduced into reaction portion 203.

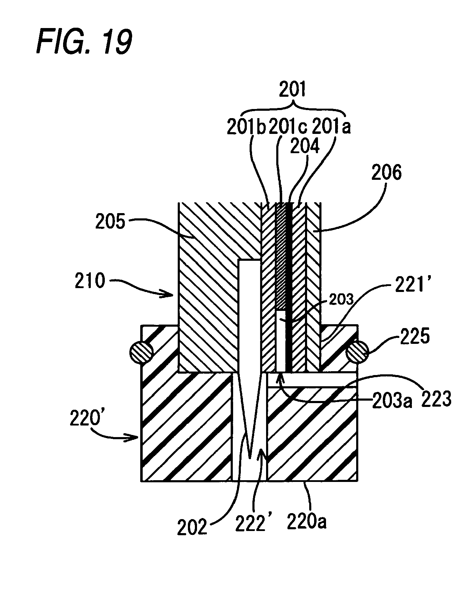

Additionally, in the above embodiments, the diameter of flow passage 222 is sized to include the puncturing needle 2 and reaction portion inlet 203a. However, the needle integral sensor of the present invention is not limited thereto. The diameter of the flow passage portion may be a diameter such that the puncturing 202 can be relatively moved. In this case, as shown in Fig. 19, groove 223 which extends to the outside of flow passage forming body 220 may be provided in the bottom face of recess 221' so that flow passage 222' and reaction portion inlet 203a communicate with each other. Groove 223 is able to serve as a ventilation passage which allows flow passage 222' and the outside of flow passage forming body 220' to communicate with each other. In such a case, if the liquid sample which has risen along flow passage 222' reaches the tip of sensor body 210 that is a ceiling surface of flow passage 222, the liquid sample flows toward groove 223 that is an air ventilation passage within flow passage 222', and is introduced into reaction portion 203 from reaction portion inlet 203a. In addition, in this case, the size of groove 223 etc. is suitably adjusted so that the liquid sample is preferentially contained into reaction portion 203 from reaction portion inlet 203a, rather than flowing out of the outside of flow passage forming body 220' through groove 223.

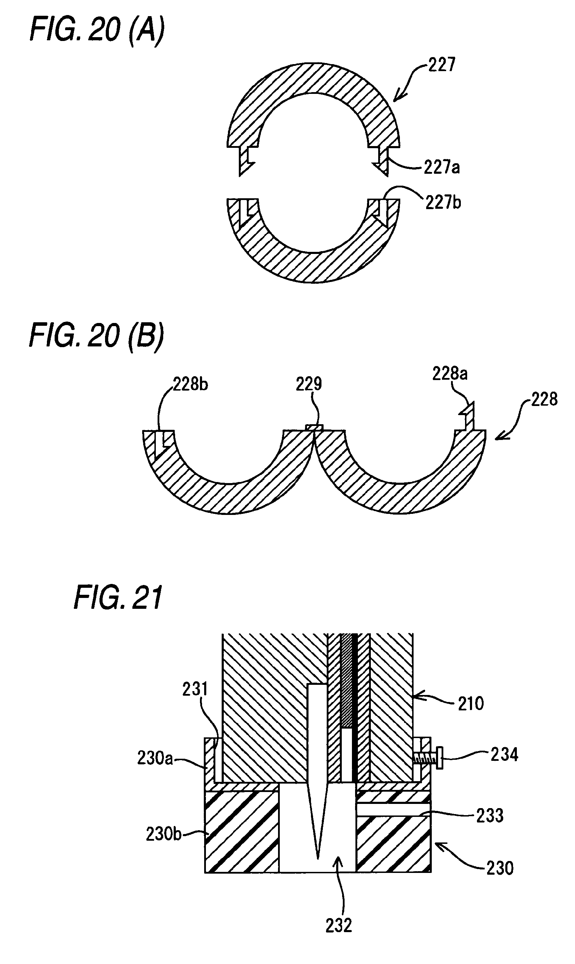

Additionally, in all the above embodiments, the C-shaped ring has been used as the fastener. However, the fastener to be used for the needle integral sensor of the present invention is not limited thereto. The fastener may be one which can fasten the outer periphery of the flow passage forming body, and reduce the diameter of the recess, for example, an O-ring having elasticity. Additionally, the fastener may be combination-type fastener 227 obtained by splitting an O-ring as shown in Fig. 20A, or fastener 228 in which semicircular portions obtained by splitting an O-ring into two are connected together by connecting tool 229, such as a hinge, as shown in Fig. 20b. In the fastener 227, engaging protrusion 227a is provided at one semicircular portion so as to protrude therefrom, and engaging recess 227b which engages engaging protrusion 227a is provided at the other semicircular portion so as to be recessed therefrom. Thus, the outer periphery of the flow passage forming body can be fastened by fitting engaging protrusion 227a into engaging recess 227b. Additionally, in fastener 228, the flow passage forming body can be fastened by attaching the semicircular portions to the outer periphery of the flow passage forming body, and fitting engaging protrusion 228a into engaging recess 228b. Moreover, the fastener is not limited to a ring which fastens the outer periphery of the flow passage forming body, but may be a cylindrical fastener which covers both the flow passage forming body and the sensor body.

Furthermore, the fastener used in the present invention is not limited to articles having a substantially ring-shaped cross-section, but may be one which only winds and fastens a corded winding body, a wire, or the like around the outer periphery. Additionally, after the outer periphery of an attachment portion is wound by a tube, a tape, etc. made of heat-shrinkable resin, the heat-shrinkable tube or tape may be shrunk by heat. Additionally, the fastener to be used in the present invention is not limited to fastening the outer periphery of an attachment portion, and reducing the diameter of the recess. A recess may be fastened from a specific direction using a fastener and the flow passage forming body may be attached and fixed to the tip of the sensor body.

Fig. 21 shows one embodiment of the needle integral sensor in which flow passage forming body 230 is attached and fixed to sensor body 210, using adjusting screw 234 as a fastener which performs fastening from a specific direction.
Flow passage forming body 230 used in the needle integral sensor shown in Fig. 21 is composed of attachment portion 230a formed of a hard material, such as plastic or metal, and flow passage portion 230b made of a viscoelastic material anchored thereto. Recess 231 recessed in attachment portion 230a is in a loose fitting state with a slightly larger size than a portion into which the tip of sensor body 210 is inserted. Flow passage forming body 230 is attached and fixed to sensor body 210 by fastening an inserted portion of sensor body 210 with adjusting screw 234 that is a fastener.

In the embodiment shown in Fig. 21, the adjusting screw is used as the fastener. However, a pin or a key other than the screw may be used as a fastener which performs fastening from a specific direction. The fastening and fixation from the specific direction by a screw, a pin, etc. is suitable in a case where a surface pressed by a screw, a pin, etc. exists in the sensor body 210, and the shape for the tip portion of sensor body 210 is capable of corresponding to fastening of suitable shapes other than a circular shape, such as a square shape or a polygonal shape. Additionally, as a female thread or pin hole corresponding to a male thread or a pin that is a fastener is recessed in the needle carrier 205 and mounting portion 206, the attachment position between sensor body 210 and flow passage forming body 230 can be fixed in a fixed position. This is useful in a case where the attachment direction of flow passage forming body 230 is determined. For example, in a case where flow passage portion 230b is provided with ventilation passage 233, and the air within flow passage 232 flows out of the outside of flow passage forming body 230 through ventilation passage 233, that is, in a case where it is expected that a liquid sample which has risen along puncturing needle 202 or a liquid sample which has risen along the inner wall surface of flow passage 232 corresponding to the opposite side of the position of reaction portion 203 with respect to needle 202 is introduced into the reaction portion 3, it is necessary to attach flow passage forming body 230 so that ventilation passage 233 is located on the side of reaction portion 203 with respect to needle 202. In such a case, positioning can be performed in the relationship between a male thread and a female thread or between a pin and a pin hole.

In addition, in all the above embodiments, the attachment position of the puncturing needle is disposed in the vicinity of the reaction portion inlet, However, the puncturing needle may be attached to the inside of the reaction portion. In this case, the electrodes are disposed so as not to contact the puncturing needle, and the reagent is applied so as to lead to the electrodes and the reaction portion. Additionally, in the above embodiments, the electrodes are provided on the insulating substrate on the side where the needle is not attached. However, in the needle integral sensor of the present invention, the positional relationship between a needle attachment position and an electrode substrate is not limited. The electrodes may be provided on a substrate on the side where the needle is attached, and a positive electrode and a negative electrode do not need to be provided in the same substrate. A negative electrode may be provided on one substrate, and a positive electrode may be provided on the other substrate. Additionally, although the detecting portion is configured such that two substrates are stuck together, for example, as disclosed in Pamphlet of PCT International Patent Publication No. 05/010519, the detecting portion may be configured such that a pair of electrodes is arranged on one substrate, and the electrodes are bent inward. As for the shape of the sensor body, the shape of the needle carrier and the mounting portion may be suitably designed according to the shape of the detecting portion. Moreover, the shape of the recess in the flow passage forming body may be suitably designed in combination therewith, and the kind of the fastener may be suitably selected. In addition, although the biosensor cartridge and needle integral sensor according to the present invention are expressed differently, they are common in that the puncturing needle and the biosensor chip are integrated. Additionally, both the flow passage forming body and the elastic body are common to each other in that they are disposed at the puncturing needle and the tip of the biosensor chip.

### INDUSTRIAL APPLICABILITY

As described above, in the biosensor cartridge according to the present invention, the tip portion of the biosensor chip body is provided with the elastic body. Thus, when the tip portion of the biosensor chip body is pushed against a subject, the subject can be punctured, and when a pushing force is weakened, the puncturing tool is pulled out of the subject by the restoring force of the elastic body, and a sample flows out of the puncture hole. Additionally, the cover member is provided around the biosensor chip body. Thus, when puncturing is performed by the thin plate-shaped biosensor cartridge, the biosensor chip body can be prevented from deforming, and puncturing can be accurately performed in a desired puncturing position. Moreover, since the cover member is provided, even when the biosensor cartridge is mounted on the driving unit, contact area is large, and the mounting is easy. Additionally, since the area of contact with the elastic body becomes large, setting to the elastic body is easy, and the problem that the elastic body is separated from the biosensor cartridge can be solved.
That is, the biosensor cartridge according to the present invention is useful as a biosensor cartridge which conducts measurement and analysis of a chemical substance using a reagent contained in a hollow reaction portion of, for example, a biosensor chip.
Additionally, in the method of manufacturing a biosensor cartridge according to the present invention, a first fitting portion provided in the cover member, and a second fitting portion provided in the biosensor chip are made to fit to each other, and thereby integrated. Thus, assembling is easy, integration can be firmly achieved, and the puncturing tool provided in the cover member can be easily attached to the biosensor chip. Additionally, the manufacturing method is useful as a biosensor cartridge which conducts measurement and analysis of a chemical substance using a reagent contained in a hollow reaction portion of, for example, a biosensor chip.

Although the present invention has been described in detail with reference to the specific embodiments, it is clear to those skilled in the art that various alternations and modifications can be made without departing from the spirit and scope of the present invention. The present application is based on Japanese Patent Application (JP2006-304972) filed on November 10, 2006, Japanese Patent Application (JP2006-313466) filed on November 20, 2006, and Japanese Patent Application (2007-043034) filed on February 22, 2007, the entire contents of which are incorporated herein by reference.

## Claims

1. A biosensor cartridge comprising:
a biosensor chip,
a puncturing tool fixed to a portion of the biosensor chip and protruding from the tip of the biosensor chip,
an elastic body provided at the tip of the biosensor chip, and
a cover member provided around the biosensor chip.

2. The biosensor cartridge of Claim 1, wherein
the elastic body and the cover member are bonded together.

3. The biosensor cartridge of Claim 1 or 2, wherein
the elastic body forms an enclosed space so as to contain a sample collection opening provided at the tip of the biosensor chip and a puncture hole formed in a subject by the puncturing tool.

4. The biosensor cartridge of any one of Claims 1 to 3, wherein
the elastic body has stickiness.

5. The biosensor cartridge of any one of Claims 1 to 4, wherein
puncturing is performed by compressing the elastic body to make the puncturing tool protrude from the tip of the elastic body.

6. A biosensor device comprising:
the biosensor cartridge of any one of Claims 1 to 5, and
a measuring instrument which is connected to detecting electrodes of the biosensor cartridge and obtains information on a sample which has been collected.

7. The biosensor device of Claim 6, further comprising:
a driving unit for puncturing, and
a connector for attaching the biosensor chip, the connector being attached to the driving unit.

8. The biosensor device of Claim 7, wherein
the cover member and the connector fit to each other.

9. A sample collecting method comprising the steps of:
performing puncturing using the biosensor cartridge of any one of Claims 1 to 5, and
collecting a sample from the sample collection opening using a suction unit connected to the biosensor cartridge.

10. A method of manufacturing a biosensor cartridge including a biosensor chip having a sample collection opening at a tip face thereof and a puncturing tool fixed to a tip portion of the biosensor chip and having a tip portion protruding therefrom,
the method comprising the step of:
making a first fitting portion provided in a cover member having the puncturing tool integrally and a second fitting portion provided in the biosensor chip fit to each other.

11. The method of manufacturing a biosensor cartridge of Claim 10, wherein
the first fitting portion is a fitting hole provided in the cover member, and
the second fitting portion is the biosensor chip body which makes the biosensor chip fit to the fitting hole.

12. The method of manufacturing a biosensor cartridge of Claim 10 or 11, wherein
the first fitting portion is either a protrusion or a recess provided along an insertion direction in the cover member, and
the second fitting portion is the other of a protrusion and a recess which fits to either the protrusion or the recess provided in the biosensor chip.

13. The method of manufacturing a biosensor cartridge of any one of Claims 10 to 12, wherein
the biosensor chip and the puncturing tool are fixed together by joining the biosensor chip and the cover member with an adhesive.

14. The method of manufacturing a biosensor cartridge of any one of Claims 10 to 13, wherein
the cover member includes a locking claw in the vicinity of a rear end in the insertion direction, and
the biosensor chip and the puncturing tool are fixed together by locking a stepped portion provided in the biosensor chip.

15. A needle integral sensor comprising:
a needle integral sensor body integrally having a puncturing needle which discharges a liquid sample from a subject, a reaction portion which contains the liquid sample, and a detecting portion which detects the result of the reaction portion, and
a flow passage forming body having an attachment portion in which a recess into which the tip of the needle integral sensor body is inserted is recessed, and a flow passage portion which forms a flow passage from an abutting portion of the subject to the reaction portion and in which the puncturing needle is inserted into the flow passage, wherein
the flow passage forming body is fastened and fixed to the needle integral sensor body by a fastener.

16. The needle integral sensor of Claim 15, wherein
at least the flow passage portion of the flow passage forming body is formed of an elastic body or a viscoelastic body.

17. The needle integral sensor of Claim 15 or 16, wherein
the attachment portion is substantially cylindrical, and
the fastener reduces an internal diameter of the recess.

18. The needle integral sensor of Claim 15 or 16, wherein
the needle integral sensor body is loosely fitted into the recess, and
the fastener fastens the needle integral sensor body from a predetermined direction.
